(19) Europäisches Patentamt / European Patent Office / Office européen des brevets

(11) **EP 3 367 098 A1**

(12) **EUROPEAN PATENT APPLICATION**

(43) Date of publication:
29.08.2018 Bulletin 2018/35

(51) Int Cl.:
*G01N 33/569* (2006.01)     *G01N 33/574* (2006.01)

(21) Application number: **17157806.5**

(22) Date of filing: **24.02.2017**

| | |
|---|---|
| (84) Designated Contracting States:<br>**AL AT BE BG CH CY CZ DE DK EE ES FI FR GB<br>GR HR HU IE IS IT LI LT LU LV MC MK MT NL NO<br>PL PT RO RS SE SI SK SM TR**<br>Designated Extension States:<br>**BA ME**<br>Designated Validation States:<br>**MA MD** | (71) Applicant: **CeMM - Forschungszentrum für<br>Molekulare Medizin GmbH<br>1090 Wien (AT)**<br><br>(72) Inventor: **KRALL, Nikolaus<br>3644 Emmersdorf a.d. Donau (AT)**<br><br>(74) Representative: **Vossius & Partner<br>Patentanwälte Rechtsanwälte mbB<br>Siebertstrasse 3<br>81675 München (DE)** |

(54) **METHODS FOR DETERMINING INTERACTION BETWEEN BIOLOGICAL CELLS**

(57)     The present invention relates to methods for determining the propensity of cells to interact in a population of cells comprising at least two distinguishable subpopulations of cells. In addition, the present invention provides methods for diagnosing a disease or predisposition to a disease of a cell donor, wherein the method comprises the determination of the propensity of cells to interact in a population of cells obtained from the donor, wherein the population of cells comprises at least two distinguishable subpopulations of cells. The invention also provides methods for determining whether a subject suffering from or predisposed to a disease will respond or is responsive to treatment with a therapeutic agent by determining the alteration of the propensity of cells to interact in a population of cells obtained from the subject, wherein the population of cells comprises at least two distinguishable subpopulations of cells. The invention also relates to methods for screening for a therapeutic agent comprising the determination whether one or more test substances alter the propensity of cells to interact.

**Description**

The present invention relates to methods for determining the propensity of cells to interact in a population of cells comprising at least two distinguishable subpopulations of cells. In addition, the present invention provides methods for diagnosing a disease or predisposition to a disease of a cell donor, wherein the method comprises the determination of the propensity of cells to interact in a population of cells obtained from the donor, wherein the population of cells comprises at least two distinguishable subpopulations of cells. The invention also provides methods for determining whether a subject suffering from or predisposed to a disease will respond or is responsive to treatment with a therapeutic agent by determining the alteration of the propensity of cells to interact in a population of cells obtained from the subject, wherein the population of cells comprises at least two distinguishable subpopulations of cells. The invention also relates to methods for screening for a therapeutic agent comprising the determination whether one or more test substances alter the propensity of cells to interact.

The understanding of cell behavior on a global scale after perturbation with diverse stimuli such as biological agents and small molecules is significantly furthered by analysis at the single-cell level, including the analysis of cell-to-cell relationships. The field of high-content imaging is continually providing evidence that cell-to-cell variability, and cell-microenvironment, in the detailed analysis of cell-population phenotypes is necessary for the determination of rare events and complete understanding of the population level phenotypes (reviewed in Snijder et al. (2011) Nature Reviews 12, 119). Variations in cellular phenotype on a global level are largely determined by the inherent properties of developing cell populations that create specialized niche microenvironments, including cell densities, cell-cell contacts, relative location and cell-space. Moreover, at the single-cell level, the heterogeneity of cellular-responses to the same perturbation can be characterized (Slack et al. (2008) PNAS 105(49): 19306-11). The complexity of cellular heterogeneity (in reference to the investigation of degree to which cancer cells react to anti-cancer drugs) reveals functional significance to the broad effect patients may have on a cellular level during therapy. However, studies which have laid the groundwork for population-characteristic analysis driven by sub-cellular and single-cell resolution have relied on genetically identical cell lines, which are not physiologically relevant to human health and disease and lack intra-population communication that occurs normally within life to further cellular and pan-cellular processes.

The immune system is regulated in two ways: 1) by soluble signaling molecules and 2) the physical interaction of cells. Examples of soluble signaling molecules that regulate the immune system are cytokines, chemokines but also include small molecules such as adenosine. Examples where cell-cell interactions regulate immune function are cell-surface bound ligands interacting with cell surface receptors such as the interaction of MHC with the T-cell receptor or PD-1 with its ligands PD-L1, both of which are necessary to elicit targeted responses or control responses. Furthermore, soluble factors can interact with receptors on two different target cells to bring them within a close spatial proximity, and induce a signaling event. Examples include antibodies that may bind to Fc receptors on one cell, and recognize a target antigen on another. Specifically, this is the case when it comes to immunomodulatory drugs and the new field of biologics such as Blinatumomab (bi-specific antibody; CD19/CD3) or rituximab (anti-CD20 antibody). Whilst soluble factors can act at a distance, cell-surface bound regulatory molecules require cells to come into close spatial proximity for their action. This latter factor, the physical interaction of immune cells through receptor mediated signaling, is imperative to mounting an elicit, directed, and strong immune response, and cell clearance / programed cell death. Further, cell-cell contact communication is the basis of the mechanisms of action of biological immunomodulatory drugs, as explained above.

There is a plethora of well-established tools to measure the concentration of soluble signaling factors such as ELISA, ELISPOT and alpha screen to assess immune function, or activity. The tracking of soluble factors involved in immune regulation is used routinely in the diagnostics of a disease and other health related biological sciences. However, there is currently only limited ability, if at all, to determine immunomodulation at the cell-cell contact level as modified by biologics, drugs, or other small compounds in a high-throughput and robust manner. The ability to systematically measure cell-cell contacts and how they are altered by perturbing the system with molecules of interest can thus reveal if a certain molecule has immunomodulatory properties or not. Conversely, when challenging a system with molecules of known immunomodulatory effects observing the effect as changes in cell-cell contacts can reveal information about the state of the model system.

A method for determining interactions of subcellular organelles has been proposed by Helmuth et al. (2010) BMC Bioinformatics 2010, 11: 372. The method derives a statistical framework for analyzing the attraction between two sets of subcellular objects X and Y. However, as used therein, subcellular attraction is biologically different from cell-cell contacts as used herein. Moreover, Helmuth et al. define a "nearest neighbor colocalization measure" between objects called Ct and then use this to generalize to the analysis of nearest neighbor distance distributions for large numbers of objects analyzed. This method is inappropriate as described by Helmuth et al. when applied to PBMCs/complex cell mixtures with the goal to detect cell-cell contacts as the number of interacting cells typically is a small subset of all cells. The nearest neighbor distribution analysis as used by Helmuth et al. would easily overlook a small subset of interacting cells, and focus on overall clustering or attraction between the two subsets of cells. Further, their technical solution is to compare observed nearest neighbor distance distributions between objects X and Y p(d) to the relative frequency of possible distances q(d) from objects Y ignoring the fact that other objects may also occupy space in the biological system. The approach thus does not correct for overall clustering tendencies. However, in cell-cell contact analysis of two subsets of PBMCs or complex cell mixtures, overall clustering is critical to correct for, as this is largely determined by experimental factors, not by biological factors of interest.

In view of the foregoing, there is a need for means and methods that allow high-throughput and cost-efficient determination of cell-cell interactions in biological samples, in particular samples comprising PBMCs or bone-marrow cells.

Thus, the technical problem underlying the present invention is the provision of improved means and methods for determining the propensity of cells to interact.

The technical problem is solved by provision of the embodiments characterized in the claims.

Accordingly, the present invention relates to methods for determining the propensity of cells to interact in a population of cells comprising at least two distinguishable subpopulations of cells, wherein the method comprises the following steps (a) determination of the number of interactions between cells of a first distinguishable subpopulation and cells of a second distinguishable subpopulation; (b) determination of the number of interactions in the population of cells by randomly assigning cells comprised in the population of cells to first and second distinguishable subpopulation of cells, wherein the absolute number of cells in the first and second distinguishable subpopulation of cells, respectively, are identical to the number of cells in the first and second distinguishable subpopulation of cells in (a); and (c) determination of the propensity of cells to interact by dividing (a) through (b), wherein propensity increases with the resulting number. In a preferred embodiment, step (b) is repeated and the determined number of interactions is averaged, preferably wherein step (b) is repeated more than at least 1000 times, at least 2000 times or at least 3000 times.

The invention furthermore relates to a population of cells obtained from a cell donor for use in determining whether the cell donor suffers from a disease or is predisposed to suffer from a disease, wherein the determination comprises the determination of the propensity of cells in the population to interact, wherein the population comprises at least two distinguishable subpopulations of cells and wherein the method comprises (a) determination of the number of interactions between cells of a first distinguishable subpopulation and cells of a second distinguishable subpopulation; (b) determination of the number of interactions in the population of cells by randomly assigning cells comprised in the population of cells to first and second distinguishable subpopulation of cells, wherein the absolute number of cells in the first and second distinguishable subpopulation of cells, respectively, are identical to the number of cells in the first and second distinguishable subpopulation of cells in (a); (c) determination of the propensity of cells to interact by dividing (a) through (b), wherein propensity increases with the resulting number; and (d) determination whether the cell donor has a disease or is predisposed to have a disease based on the propensity of cells to interact.

The invention also provides for a method for diagnosing a disease or predisposition to a disease of a cell donor, wherein the method comprises the determination of the propensity of cells to interact in a population of cells obtained from the donor, wherein the population of cells comprises at least two distinguishable subpopulations of cells and wherein the method comprises (a) determination of the number of interactions between cells of a first distinguishable subpopulation and cells of a second distinguishable subpopulation; (b) determination of the number of interactions in the population of cells by randomly assigning cells comprised in the population of cells to first and second distinguishable subpopulation of cells, wherein the absolute number of cells in the first and second distinguishable subpopulation of cells, respectively, are identical to the number of cells in the first and second distinguishable subpopulation of cells in (a); (c) determination of the propensity of cells to interact by dividing (a) through (b), wherein propensity increases with the resulting number; and (d) determination whether the cell donor suffers from a disease or is predisposed to suffer from a disease based on the propensity of cells to interact.

The invention also relates to a method for determining whether a subject suffering from or predisposed to a disease will respond or is responsive to treatment with a therapeutic agent by determining the alteration of the propensity of cells to interact in a population of cells obtained from the subject, wherein the population of cells comprises at least two distinguishable subpopulations of cells and wherein the method comprises (a) determination of the number of interactions between cells of a first distinguishable subpopulation and cells of a second distinguishable subpopulation; (b) determination of the number of interactions in the cell sample by randomly assigning cells comprised in the population of cells to first and second distinguishable subpopulation of cells, wherein the absolute number of cells in the first and second distinguishable subpopulation of cells are identical to the number of cells in the first and second distinguishable subpopulation of cells in (a); (c) determination of the propensity of cells to interact by dividing (a) through (b), wherein propensity increases with the resulting number; and (d) determination of the alteration of the propensity of cells to interact by comparing the propensity prior to addition of a therapeutic agent and subsequent to addition of a therapeutic agent and thereby determining whether the subject will respond or is responsive to treatment with the therapeutic agent.

The invention also relates to a population of cells obtained from a cell donor for use in a diagnostic method for determining whether a subject suffering from or predisposed to a disease will respond or is responsive to treatment with a therapeutic agent, wherein the determination comprises the determination of the propensity of cells in the monolayer to interact, wherein the monolayer comprises at least two distinguishable subpopulations of cells and wherein the method comprises (a) determination of the number of interactions between cells of a first distinguishable subpopulation and cells of a second distinguishable subpopulation; (b) determination of the number of interactions in the cell sample by randomly assigning cells comprised in the cell sample to first and second distinguishable subpopulation of cells, wherein the absolute number of cells in the first and second distinguishable subpopulation of cells, respectively, are identical to the number of cells in the first and second distinguishable subpopulation of cells in (a); (c) determination of the propensity of cells to interact by dividing (a) through (b), wherein propensity increases with the resulting number; and (d) determination of the alteration of the propensity of cells to interact by comparing the propensity prior to addition of a therapeutic agent and subsequent to addition of a therapeutic agent and thereby determining whether the subject will respond or is responsive to treatment with the therapeutic agent.

The invention furthermore provides a method for screening of a therapeutic agent by determining the alteration of the propensity of cells to interact in a population of cells due to addition of one or more test substance(s), wherein the population of cells comprises at least two distinguishable subpopulations of cells and wherein the method comprises (a) determination of the number of interactions between cells of a first distinguishable subpopulation and cells of a second distinguishable subpopulation; (b) determination of the number of interactions in the cell sample by randomly assigning cells comprised in the cell sample to first and second distinguishable subpopulation of cells, wherein the absolute number of cells in the first and second distinguishable subpopulation of cells, respectively, are identical to the number of cells in the first and second distinguishable subpopulation of cells in (a); (c) determination of the propensity of cells to interact by dividing (a) through (b), wherein propensity increases with the resulting number; and (d) determination of the alteration of the propensity of cells to interact by comparing the propensity prior to addition of one or more test substance(s) and subsequent to addition of one or more test substance(s) and thereby determining whether the one or more test substance(s) qualify as therapeutic agent.

The invention furthermore relates to a population of cells for use in screening of a therapeutic agent by determining the alteration of the propensity of cells to interact in the population of cells due to addition of one or more test substance(s), wherein the population of cells comprises at least two distinguishable subpopulations of cells and wherein the method comprises the following steps (a) determination of the number of interactions between cells of a first distinguishable subpopulation and cells of a second distinguishable subpopulation; (b) determination of the number of interactions in the cell sample by randomly assigning cells comprised in the cell sample to first and second distinguishable subpopulation of cells, wherein the absolute number of cells in the first and second distinguishable subpopulation of cells, respectively, are identical to the number of cells in the first and second distinguishable subpopulation of cells in (a); (c) determination of the propensity of cells to interact by dividing (a) through (b), wherein propensity increases with the resulting number; and (d) determination of the alteration of the propensity of cells to interact by comparing the propensity prior to addition of one or more test substance(s) and subsequent to addition of one or more test substance(s) and thereby determining whether the one or more test substance(s) qualify as therapeutic agent.

In one embodiment first and second distinguishable subpopulations are identical.

In a preferred embodiment of the invention, step (b) is repeated and the determined number of interactions is averaged, preferably wherein step (b) is repeated more than at least 1000 times, at least 200 times, at least 3000 times.

In a preferred embodiment of the invention the cells used in the methods of the invention or in the population of cells of the invention are peripheral blood mononuclear cells (PBMCs) or bone marrow cells.

The disease to be diagnosed in the present invention is preferably a myeloproliferative disorder, inflammatory disorder, latent virus infection, cellular growth disorder, cellular chemotaxis disorder, metabolic disorder or autoimmune disorder; or leukemia or lymphoma.

Thus, the invention relates to methods for quantifying the intrinsic propensity of cells to interact with each other, i.e., the physical interaction of cells, in particular of peripheral blood mononuclear cells (PBMC), bone marrow, or other multi-lineage primary mammalian material such as any mononuclear cell containing sample taken for, e.g., diagnostic purposes as detected in images of the material. Preferably, the methods of the invention are performed using cell samples comprising a monolayer of cells and suitable imaging techniques such as confocal microscopy. The cells comprised in the sample to be analyzed, e.g. the monolayer, may be identified using fluorescent dyes or other markers known in the art and thus distinguished. Accordingly, the term "distinguishable subgroup" as used herein refers to cells that are part of a larger population and can be distinguished from other cells in the population by means of a cell marker. That is, cells of two distinguishable subgroups may belong to the same or different cell type as long as the cells show differing expression profiles of cell markers, which makes them distinguishable using imaging techniques such as confocal microscopy. In order to easily determine whether cells belong to a subgroup of cells, it is preferred to provide cells in form of a monolayer. As shown in the appended Examples, monolayer formation can be done using a method known in the art, preferably the method as taught in WO 2016/046346. Accordingly, in a preferred embodiment of the invention, the methods of the invention further comprise, prior to step (a), the formation of a monolayer comprising the cells of the cell sample used.

In the present invention, the propensity of cells to interact is expressed as an interaction score. Thus, the propensity of cells to interact that is due to biological causes such as expression of adherence molecules, receptor-ligand pairs and other cell-derived factors that mediate cell-cell interactions or agents that physically crosslink cells such as certain antibodies and bispecific antibodies or biologicals as opposed to extrinsic factors influencing cell-cell interactions such as cell density and abundance, is expressed as an interaction score. Accordingly, the present invention also relates to how an interaction score, as a measure of the propensity of cells to interact with each other, can determine a response, or lack of response, of a disease and/or healthy sample to a therapeutic agent and/or drug. The invention further relates to the use of the interaction score for diagnosing a disease, or predisposition to a disease, in a patient and/or donor. The invention also provides methods using the interaction score to determine whether a disease is likely to respond or is responsive to treatment with a therapeutic agent, and/or to find and assess the mechanism and effect of potential new therapeutic agents or drugs (screening methods). In this regard, methods of the invention to assess whether diseased cells are responsive, i.e. their interaction score changes, to a therapeutic agent/drug and/or a chemical substance that is to be assessed as potential novel therapeutic agent/drug generally rely on the use of a reference sample. This reference sample, in regards to assaying if healthy or diseased cells respond (i.e., the interaction score changes), is compared to a reference control stimulation, such as a stimulation with the vehicle DMSO or PBS. If the interaction score is being used to determine the predisposition or diagnosis of a disease then the reference sample is a healthy donor. Accordingly, in a preferred embodiment, a sample obtained from a reference donor is used in the diagnostic methods provided herein and in the methods for determining whether a donor is likely to respond to treatment. The interaction score as defined herein analyses the location of cells (i.e., spatial layout) in images for, but not limited to, 1) immunomodulatory drug discovery (i.e., drugs or other therapeutics that change the properties and/or behavior of cells of the immune system to gain the warranted outcome), 2) defining the mechanism of action of known immunomodulatory therapeutics, 3) defining the susceptibility or function of a drug in a patient sample *ex vivo.* Accordingly, the novel and inventive methods of the invention can be used in combination with imaging techniques, for example, imaging of a monolayer of cells, in particular PBMCs or bone marrow cells. The methods of the invention can also be used to determine the relationship of cells to each other and how relationship changes after incubation with chemical substances such as small molecules, biologics or other soluble factors. The methods of the invention can also be used to determine changes in populations comprised in the cell sample. Moreover, the methods of the invention can be used to determine a predisposition to a disease.

Thus, the invention relates to, *inter alia,* methods for diagnosing a disease or predisposition to a disease of a cell donor, wherein the method comprises the determination of the propensity of cells to interact in a population of cells obtained from the donor, wherein the population of cells comprises at least two distinguishable subpopulations of cells and wherein the method comprises the following steps (a) determination of the number of interactions between cells of a first distinguishable subpopulation and cells of a second distinguishable subpopulation; (b) determination of the number of interactions in the population of cells by randomly assigning cells comprised in the population of cells to first and second distinguishable subpopulation of cells, wherein the absolute number of cells in the first and second distinguishable subpopulation of cells, respectively, are identical to the number of cells in the first and second distinguishable subpopulation of cells in (a); (c) determination of the propensity of cells to interact by dividing (a) through (b), wherein propensity increases with the resulting number; and (d) determination whether the cell donor suffers from a disease or is predisposed to suffer from a disease based on the propensity of cells to interact. In a preferred embodiment, step (b) is repeated and the determined number of interactions is averaged, preferably wherein step (b) is repeated more than at least 1000 times, at least 2000 times or at least 3000 times.

The methods of the present invention can also be used to determine and predict the therapeutic value of a drug or treatment by quantifying the physical interaction of cells to each other. Thus, the present invention also relates to methods for determining whether a subject suffering from or predisposed to a disease will respond or is responsive to treatment with a therapeutic agent by determining the alteration of the propensity of cells to interact in a population of cells obtained from the subject, wherein the population of cells comprises at least two distinguishable subpopulations of cells and wherein the method comprises the following steps (a) determination of the number of interactions between cells of a first distinguishable subpopulation and cells of a second distinguishable subpopulation; (b) determination of the number of interactions in the cell sample by randomly assigning cells comprised in the population of cells to first and second distinguishable subpopulation of cells, wherein the absolute number of cells in the first and second distinguishable subpopulation of cells are identical to the number of cells in the first and second distinguishable subpopulation of cells in (a); (c) determination of the propensity of cells to interact by dividing (a) through (b), wherein propensity increases with the resulting number; and (d) determination of the alteration of the propensity of cells to interact by comparing the propensity prior to addition of a therapeutic agent and subsequent to addition of a therapeutic agent and thereby determining whether the subject will respond or is responsive to treatment with the therapeutic agent. In a preferred embodiment, step (b) is repeated and the determined number of interactions is averaged, preferably wherein step (b) is repeated more than at least 1000 times, at least 2000 times or at least 3000 times.

As shown in the appended Examples, the methods of the present invention provide a unique screening system that can be used, *inter alia,* in biological, biochemical and biophysical research. Moreover, the methods of the present invention can be used in medical diagnostic and screening methods, *e.g.* in automated medical diagnostic and screening methods; see Example 5. The methods provided herein require minimal donor material, *e.g.,* cell numbers as obtained for routine diagnostic analysis protocols may be used to test or analyze greater numbers of perturbations (*e.g.,* individual test conditions) per donation than is possible using current methods known in the art. Specifically, in a preferred embodiment, the methods of the present invention use and/or the population of cells of the invention is in the form of a monolayer of cells formed by using cells that are cultured in a culture device at a density of about 100 cells per $mm^2$ growth area to about 30000 cells per $mm^2$ growth area. More preferably, at a density of about 500 cells per $mm^2$ growth area to about 20000 cells per $mm^2$ growth area, about 1000 cells per $mm^2$ growth area to about 10000 cells per $mm^2$ growth area, about 1000 cells per $mm^2$ growth area to about 5000 cells per $mm^2$ growth area, or about 1000 cells per $mm^2$ growth area to about 3000 cells per $mm^2$ growth area. Most preferably the cells are cultured at a density of about 2000 cells per $mm^2$ growth area. Culturing cells at the above densities leads to formation of a monolayer of cells, which may be stained, fixed and/or imaged prior to analysis using the methods of the present invention.

The term "cell-cell interactions" as used herein refers to, *inter alia,* the direct interactions between cell surfaces that play a crucial role in the development and function of multicellular organisms. These interactions allow cells to communicate with each other in response to changes in their microenvironment. Such cell-cell interactions can be stable such as those made through cell junctions. These junctions are involved in the communication and organization of cells within a particular tissue. Others are transient or temporary such as those between cells of the immune system or the interactions involved in tissue inflammation. These types of intercellular interactions are distinguished from other types such as those between cells and the extracellular matrix.

In the methods of the present invention and/or in the population of cells of the present invention, natural occurring cell-cell interactions are preferably maintained during formation of the representation of cells that is to be analyzed. That is, during formation of the monolayers of cells used in a preferred embodiment of the present invention, cell-cell interactions as defined herein are maintained. Maintaining cell-cell interactions means that a cell interacting with another cell in a natural environment will also interact with said other cell or a cell of the same cell type prior to the methods and during formation of the monolayers provided herein. That is, the overall cell-cell interactions are maintained, while cells do not necessarily maintain interaction with the same interacting cell. Accordingly, the methods provided herein provide for a model system that represents a physiologically relevant status of the cells to be analyzed.

The cell-cell interactions described above are preferably maintained during the formation of the representation of cells used in the methods of the invention and formed by the population of cells of the invention, thus, preferably in the monolayer formed prior to the methods of the present invention. Generally, cell-cell interactions naturally occur in a sample comprising, for example, PBMCs or bone-marrow cells or other cells. The person skilled in the art can determine whether cell-cell interactions are naturally-occurring and are maintained by the cells during sample formation, preferably in the monolayer. In particular, the person skilled in the art can use methods well-known in the art. In particular, the cell-cell interactions are maintained during addition of detectable labels and/or dyes, in particular a viability dye. Subsequent to formation of the cell sample, preferably the monolayer, cell-cell interactions may be interrupted by, e.g, fixation of the cells, preferably the monolayer prior to imaging.

Subsequent to formation of the representation of cells, preferably the monolayer and when performing the methods of the present invention, cell-cell interactions may be interrupted or newly built by addition of chemical substances, e.g. therapeutic agents. In the methods of the present invention, such changes/alterations may be used to determine whether chemical substances, therapeutic agents, have an impact on cell-cell interactions, which may be predictive of therapeutic responses. Additionally, or alternatively, changes/alterations may be used to screen novel therapeutic agents/drugs, e.g. from a library of chemical substances.

Cell-cell interactions maintained during the formation of the representation of cells used in the methods of the invention, preferably in the monolayer, and maintained prior to the methods of the present invention include, for example, the following cell-cell interactions, in particular in PBMCs, bone marrow, or other material containing mononuclear cells, indicative of specific diseases and/or indicative of healthy donors.

Table 1

| Disease | Interaction | Function | Marker 1 | Marker 2 | Effect on interaction propensity |
|---|---|---|---|---|---|
| Inflammation (e.g. gout) / infection (e.g. EBV) | T-cell - B-cell | Antigen presentation, T-cell function activity | CD3 | CD19 | increases |

(continued)

| Disease | Interaction | Function | Marker 1 | Marker 2 | Effect on interaction propensity |
|---|---|---|---|---|---|
| Inflammation (e.g. gout) / infection (e.g. EBV) | T-cell - DC | T-cell activation | CD3 | CD11C | increases |
| Inflammation (e.g. gout) / infection (e.g. EBV) | T-cell - Macrophage | Antigen presentation | CD3 | CD14 | increases |
| Inflammation (e.g. gout) / infection (e.g. EBV) / wound healing | T-cell - epithelial cell | T-cell invasion into tissue | CD3 | EpCAM | increases |
| Cancer | T-cell - circulating cancer cell (cancer defined as not healthy; metastasized) | Cytotoxicity | CD3 | CD34 | increases |
| Inflammation (e.g. gout) / infection (e.g. EBV) | T-cell - NK cell | Adaptive immune response promotion | CD3 | CD56 | increases |
| Inflammation (e.g. gout) / infection (e.g. EBV) / humoral antibody response against a pathogen | B-cell - DC | B-cell activation | CD19 | CD11c | increases |
| Inflammation (e.g. gout) / infection (e.g. EBV) | B-cell - macrophage | B-cell activation | CD19 | CD14 | increases |
| Inflammation (e.g. gout) / infection (e.g. EBV) / humoral antibody response against a pathogen / autoimmune disease (e.g. lupus) | B-cell-epithelial cell | B-cell activation | CD19 | EpCAM | increases |
| Cancer | B-cell - cancer cell | Potential antibody-directed anti-cancer response | CD19 | CD34 | increases |
| Inflammation (e.g. gout) / infection (e.g. EBV) | B-cell - NK cell | Immune propagation | CD19 | CD56 | increases |
| Inflammation (e.g. gout) / infection (e.g. EBV) | DC - macrophage | Immune propagation | CD11c | CD14 | increases |
| Inflammation (e.g. gout) / infection (e.g. EBV) | DC - epithelial cell | Immune propagation | CD11c | EpCAM | increases |
| Cancer | DC - cancer cell | Anti-cancer responses | CD11c | CD34 | increases |
| Cancer | macrophage - cancer cell | Anti-cancer responses | CD14 | CD34 | increases |
| Inflammation (e.g. gout) / infection (e.g. EBV) | macrophage - epithelial cell | Immune propagation / tissue repair | CD14 | EpCAM | increases |

(continued)

| Disease | Interaction | Function | Marker 1 | Marker 2 | Effect on interaction propensity |
|---|---|---|---|---|---|
| Inflammation (e.g. gout) / infection (e.g. EBV) | macrophage - NK cell | Immune propagation | CD14 | CD56 | increases |
| Cancer | T-cell - tumor cell | Anti-cancer responses | CD3 | CD56 | increases |
| Cancer | NK-cell - tumor cell | Anti-cancer responses | CD56 | CD56 | increases |
| Inflammation (e.g. gout) / infection (e.g. EBV) | monocyte - monocyte | | CD14 | CD14 | increases |
| Gamma chain deficiency | T-cell - dendritic cell | T-cell activation | CD3 | CD11c | decreases |

Accordingly, naturally-occurring cell-cell interactions can be determined/assessed/detected using cell markers. Cell markers are proteins expressed by a cell of a particular type that alone or in combination with other proteins allow cells of this type to be distinguished from other cell types. That is, by using cell markers expressed on the surface of cells or within (including within the cytoplasm or within an internal membrane) cells comprised in the population of cells of the invention, e.g. comprised in a sample obtained from a donor, cells comprised in the population of cells can be distinguished. Accordingly, the two or more distinguishable subgroups of cells are not limited to cells belonging to different cell types. Rather, cells of the two or more distinguishable subgroups may be of the same cell type as long as the subgroups are distinguishable using cell markers, e.g. those expressed on their surface, e.g. cells of the same cell type at different disease stages. That is, natural-occurring cell-cell interactions can be determined/assessed/detected using markers shown in the above Table. For example, natural-occurring cell-cell interactions indicative of a healthy donor can be detected using, *inter alia,* cell marker pairs for CD11c and CD3, CD14 and CD3, CD11C and CD8, CD19 and CD3. However, in one embodiment of the invention first and second distinguishable subpopulations are identical.

Where cells comprised in the representation of cells, e.g. the population of cells of the invention, preferably in the monolayer are detected/labeled using the above provided cell markers and to measure the cellular interaction propensity of those positive cells indicative of specific diseases, the associated disease can be diagnosed in the cell donor and/or treatment of the associated disease can be assessed. Also, where cells comprised in the representation of cells, e.g. the population of cells of the invention, preferably in the monolayer are detected/labeled using markers indicative of healthy donors, the natural-occurring cell-cell interactions can be assessed/determined for such healthy donors.

As discussed above, the skilled person is aware of means and methods how to determine/assess/track/verify cell-cell interactions. In particular, the person skilled in the art can distinguish between natural-occurring cell-cell interactions and those introduced during the preparation of a cell sample. As such, the skilled person understands that cells of the same type and/or cells of different types interact in a living organism. Moreover, the person skilled in the art understands that cells of distinguishable subgroups of cells comprised in the cell population interact in a living organism. Thus, the majority of cells comprised in the cell sample maintain their natural-occurring cell-cell interactions. That is, the majority of cells, in particular at least 50% of the cells comprised in the cell sample, preferably 60%, 65%, 70%, 75%, 80%, 85%, 90%, 95%, or 100% of the cells comprised in the cell sample interact with the same cell or a cell of the same cell type or a cell of the same distinguishable subgroup as *in vivo.* Cell-cell interactions can be verified/assessed/determined using methods well-known in the art. For example, confocal microscopy can be used to assess/determine/verify whether cell-cell interactions are between cells interacting in a natural environment or between cells that do not show interaction in a natural environment. Such non-natural cell-cell interactions may be due to, *inter alia,* cell clumping.

The methods of the present invention control for changes/alterations in the numbers of each distinguishable subpopulation of cells comprised in the population of cells/monolayer and the cell-cell interactions observed, and thus, controls for cytotoxicity and/or cell growth and differences in overall cell density. The methods of the invention are not equivalent to other methods of analyzing adherent cell lines as understood in the art, i.e., determining clonal densities of cells, clonal outgrowth, or the analysis of cell-line monolayer development. Rather the samples used for the methods of the invention may comprise cultures of high density comprising a majority of cells in direct contact with one or more other cells, but not necessarily adhered to the culture surface, or may comprise cultures of low density, wherein only few cells within the sample, preferably the monolayer, have direct physical contact with other cells in the sample, preferably the monolayer. The methods of the invention may be used for samples containing adherent and/or non-adherent cells of intermediate density, having discrete areas wherein cells are in contact with one or more cells and other areas where the cells exhibit no contact with other cells. As such, the methods of the invention provide a unique tool for use with a wide variety of cell samples without requiring further adaptation. Thus, the methods of the invention can be used in a broad range of applications, which is a major advantage over methods known in the art. Because the methods of the invention require no or only little further adaptation to the particular sample, the methods of the invention are more cost-efficient than methods of the prior art and more time-efficient than methods of the prior art.

Specifically, previous methods for use in analyzing cell-cell contacts in monolayers of non-adherent mononuclear cells or mixtures of adherent and non-adherent mononuclear cells derived from blood or bone marrow of diseased or healthy donors with automated microscopy have been described in the prior art, e.g. WO 2016/046346. Briefly, cells were processed into monolayers consisting of non-adherent cells or mixtures of adherent and non-adherent cells, exposed to a stimulus for a defined period of time, fixed and stained with fluorescently labeled reagents to identify cells with properties of interest such as fluorescently labeled antibodies. The novel and inventive methods described in WO 2016/046346 to provide monolayers of cells, in particular PBMCs or bone-marrow cells, are also used in the present invention in order to provide suitable imaging samples of cells to be analyzed. However, the present invention is focused on the analysis of such images rather than the provision of suitable samples. Yet, unlike in previously described methods to prepare monolayers of mononuclear cells, physiologically relevant cell-cell contacts were maintained by utilizing a system described in WO 2016/046346, which maintained a stable cell environment. Cell-cell contacts were inferred from microscopic images of monolayers by defining a threshold when using the analysis method described in WO 2016/046346. If two cells were closer than the set threshold they were deemed to be interacting, otherwise not. The number of cells in contact with other cells depends on the overall cell density within a microscopic image of mononuclear cell monolayers. Furthermore, analyzing the interactions between two clearly distinguishable cell types A and B, the number of A cells in contact with B cells also depends on the proportion of A and B cells in the total sample and their relative proportion. Simple counting of A cells in contact with B cells in a microscopic image thus holds little biological information. To measure the intrinsic propensity of two cell types A and B to interact with each other (i.e., the tendency of the cells to interact with each other that is due to biological properties associated with the cells) one needs to normalize numbers of A cells in contact with B cells to total cell density and the fraction of A and B cells in the sample. The prior art achieved this by using the formula: Obs = #A cells that neighbor at least one B cells divided by #A cells, whereby Fi = # cells with at least one neighbor divided by # all cells; Fa=#A cells divided by # all cells; and Fb = # B cells divided by # all cells, wherein "#" means "number of". Then, E = Fa * Fb * Fi and the normalized interaction score was computed as the fraction of Obs / E and typically given as the log2 of this fraction as a measure of the intrinsic propensity of cells of type A and B to interact with each other. This method of the prior art robustly normalizes for changes in cell density (Fi) and fraction of interacting cell types (Fa and Fb) when the overall cell density in the analyzed image is low (in the order of 1 interacting cell per cell) and changes in Fa and Fb over the experiments are limited. However, the score becomes increasingly inaccurate in cases of high cell densities (>> 1 interacting cell per cell) and when large changes in Fa are expected over the course of the experiment (Examples 3 and 4). This deficiency is overcome by provision of the methods of the invention. Thus, the methods of the present invention are suitable for use in a wider range of samples, in particular samples comprising high cell densities and/or samples that undergo changes of relative cell populations.

In particular, to solve this technical problem, the total number of observed interactions $I_{obs}$(A->B) between cells of type A with cells of type B are counted. A and B are two distinguishable cell subpopulations; an interaction is defined as two cells being closer than a certain threshold value and the total number of cells analyzed is the number of cells of type A plus the number of cells of type B plus the number of cells of neither type A or B.

$I_{obs}$(A-B) is then normalized to the interactions expected to occur by random $I_{rand}$(A-B) by dividing $I_{obs}$(A-B) through $I_{rand}$(A-B). Accordingly, in the methods of the present invention, the number of interactions between A and B, i.e. first and second distinguishable subpopulation, is determined by methods described herein. In a second step, the theoretically expected number of interactions between A and B, i.e. first and second distinguishable subpopulation, based on their relative occurrence is determined, by randomly assigning cells of the population to either subgroup A or B, whereby the total number of cells in each subgroup remains identical. That is, a cell A within the threshold distance to a cell B may be after being randomly assigned to A or B either still be interacting with a cell of subgroup B or not, depending on its own random assignment and that of the neighboring cell. Thus, the total number of observed interactions may be either higher than it would be expected based on randomly assigning cells to subgroup A or B or lower, depending on the propensity of cells of A and B to interact with each other. In order to reliably determine the number of interactions by randomly assigning cells to subgroup A or B, this step is preferably repeated and the results are averaged. Preferably, this step is repeated at least 1000 times, at least 2000 times or at least 3000 times. $I_{rand}$(A->B) can alternatively be computed by counting the total number of interactions $I_{tot}$ between all cells in analogy to the definition above, multiplying with the fraction of A cells of all cells being analyzed $F_A$ and the fraction of B cells of all cells being analyzed $F_B$. The interaction propensity as measured according to the present invention and as expressed as interaction score (IS') can thus be defined as:

$$IS' = \frac{I_{obs}(A \to B)}{I_{tot} * F_A * F_B}$$ (Formula I).

The threshold is defined to be a positive number. If the distance between two cells is below the threshold, they are deemed to be interacting. The same fixed threshold is defined for all cells being analyzed. Here, the choice of threshold intimately relates to the type of interaction being studied. A low threshold is more sensitive to changes in high affinity and long-term interactions. A higher threshold is also sensitive to lower affinity and thus more transient interactions. Additionally, the threshold must be set in accordance with the overall size of the cells studies. Larger cells require larger thresholds and smaller cells smaller thresholds.

For some applications, the optimal threshold leading to maximum measurement sensitivity can be determined empirically if a positive control that modulates the interaction into a defined direction is available. Here, the IS' after treatment with positive and negative control for different reasonable choices of threshold between the limits defined above are computed and the threshold giving the maximum difference between IS' under positive control conditions minus IS' under negative control conditions whilst minimizing standard deviation chosen. Typically, the threshold chosen in the methods of the present invention and for analysis of the monolayers of the present invention is in the range of about twice the diameter of a cell to be analyzed. Thus, in a preferred embodiment of the present invention, the threshold value is within the range of about 5 $\mu$m to about 35 $\mu$m, more preferably in the range of about 10 $\mu$m to about 20 $\mu$m, and even more preferably in the range of about 10 $\mu$m to about 15 $\mu$m. In a particularly preferred embodiment, the threshold is about 12 $\mu$m. That is, in a preferred embodiment, for each cell of a first distinguishable subpopulation the number of interactions is increased by the number of cells of a second distinguishable subpopulation that have a distance of less than about 12 $\mu$m to the cell of the first distinguishable subpopulation. Preferably, the distance is measured between the center of cells to be assessed.

The present invention thus provides methods to quantify the intrinsic propensity of cells to interact with each other, that, when applied to microscopic images of cells, in particular PBMCs, bone marrow, or other adherent and/or non-adherent primary mononuclear or other cell material robustly normalizes for changes in cell density and/or changes in total cell numbers of the population being analyzed and/or changes in total cell numbers of subpopulations and/or relative cell numbers of subpopulations analyzed; as shown in Example 3 and 4.

The cells, in particular PBMCs, bone marrow cells, or other adherent- and non-adherent primary cells for use in the methods of the present invention and/or for the preparation of the sample comprising the population of cells of the invention, preferably the monolayer, may be isolated from a sample obtained from a healthy subject, i.e. not suspected to suffer from a disease or suspected to be predisposed to a disease, or may be isolated from a sample obtained from a subject known to be suffering from a disease or suspected to suffer from a disease. The diagnosis of the disease state of the subject may be made by standard methods routinely performed by those skilled in the art, e.g., physicians. Such traditional methods may be supplemented or replaced with the methods of the present invention. For example, in order to determine whether a subject suffers or is likely to suffer from a disease, a cell-cell interaction pattern is determined that is characteristic for the respective disease using samples from subjects known to suffer from the disease. Additionally, or alternatively, the cell-cell interaction pattern of a healthy donor may be used to determine differences that likely are due to the respective disease. Cell interaction pattern here refers to the propensity of one or more different cell types to interact with each other determined according to the present invention.

Accordingly, the invention provides for methods for use in a diagnostic method for determining whether a disease will respond or is responsive to a therapeutic agent. For example, a cell-cell interaction pattern from a healthy donor may be used as reference and compared to the cell-cell interaction pattern determined for a diseased subject. Subsequent to addition of a therapeutic agent, changes/alterations of the cell-cell interaction pattern may be used to determine whether the overall cell-cell interaction pattern is altered/changed towards the cell-cell interaction pattern of a healthy subject used as reference. Alternatively, or additionally, subsequent to addition of a therapeutic agent, changes/alterations of the cell-cell interaction pattern may be used to determine whether the overall cell-cell interaction pattern is altered/changed towards the cell-cell interaction pattern expected from the mechanism of action of the therapeutic agent (Example 10). Accordingly, the methods provided herein enable the quantification of cellular response and activity at the single-cell, multiple-cell, and at the global level (e.g. whole-population changes) and thus provide unique systems allowing the evaluation of cellular, in particular PBMC or bone-marrow, response to one or more therapeutic agents. Thus, the methods provided herein may be, *inter alia,* used as generic models allowing evaluation of therapeutic response, or likelihood of therapeutic response, e.g., where the cells, in particular PBMCs or bone-marrow cells, are isolated from samples obtained from healthy subjects.

The therapeutic response, as determined by the methods of the present invention or by using the population of cells of the invention, preferably in the form of a monolayer of cells, of two or more samples obtained from two or more donors suffering from the same disease and/or predisposed to suffer from the same disease (or combinations thereof) may be used to develop a predictive standard, baseline, or expected response representative of a population suffering from the disease or predisposed to suffer from the disease. Alternatively, or additionally, the methods provided herein may be used in a diagnostic method to predict whether the donor providing the sample from which the cells were isolated is suffering from or predisposed to a disease and/or whether the cell donor will respond or is responsive to treatment with a therapeutic agent. "Diseases" as described within this invention can refer to hematological cancers, or hematological malignancies, solid tumor cancers, or solid tumor cancers that have disseminated into the peripheral tissue, inflammatory diseases such as arthritis and atherosclerosis (sampled from the site of diseases manifestation or from the periphery), and any other disease that where progression of said disease is mediated by cell-to-cell physical communication or physical communication driven by soluble factors.

To diagnose a disease in an individual using the method of the invention, interactions between one or more cell types in a cell sample of the individual are measured that are different in individuals suffered or predisposed to suffering from the disease and healthy individuals. Further, to determine if a patient will respond to a certain treatment using the method of the invention, the interactions between one or more cell types in a cell sample of the patient following or not following the addition of the drug of interest to the cell sample are measured that are different in individuals responding to the treatment and those not responding to the treatment.

For instance, the current method can be used for the diagnosis of a disease, such as rheumatoid arthritis, where both the diagnosis, the severity of, and the drug effect on the disease can be measured by quantifying the interaction propensity between activated B-cells (CD80+ and CD19+) to Th17 T-cells (CD3+ and CD28+) within the synovial fluid or peripheral blood. The closer the interactions of these activated cells, the higher the chance of a positive rheumatoid arthritis diagnosis. The same can be performed for a disease such as chronic reactive arthritis, under the disease category of secondary sterile inflammation post bacterial infection, can be diagnosed by quantifying the interaction of monocytes (CD14+) and T-cells (CD3+) within the synovial fluid, after clearance of the bacterial pathogen. As a last example, atherosclerosis can be diagnosed by determining the spatial interaction of inflammatory resident monocytes (CCR2-, CD16+) within the blood to patient endothelial cells (CCR5+). All of these diseases can be tracked, the severity determined, and the effect of a drug quantified using this method.

Analysis of the therapeutic response, using the methods provided herein, of the cells within the images is predictive of the response of the disease state to the therapy tested in the donor; in this respect the methods of the invention provide advantages over current methods available in the art. For example, applying the methods provided herein to images of non-adherent material form a patient with a hematological cancer, will quantify not only the spatial resolution of normal cells, but also disease cells, e.g., cells having abnormal phenotype or genotype themselves or representative of a disease state (e.g., having increased or decreased concentration relative to expected concentrations in a healthy individual).

Therefore, using the methods provided herein, the relationship of the cells within the images can be tracked after addition of the therapeutic agent; this is a quantification of the immunomodulatory ability of the therapeutic agent. And these results can determine if the therapy agent has an activity on the immune modulation, and can be translated to the clinic for patient treatment.

The majority of cells used in the methods of the present invention and comprised in the population of cells of the invention, preferably in the monolayers are found in a physiologically-relevant state, which means that preferably 60%, 70%, 80%, 90%, 95% or 100% of the cells are in a physiologically-relevant state. The above percentages of cells in a physiologically-relevant state used in the methods of the present invention and comprised in the population of cells of the invention, preferably in the monolayers are determined/measured/assessed using methods well-known in the art. In particular, whether a cell sample comprises cells found in a physiologically-relevant state is determined by quantification of cells comprised in the cell sample/monolayer. This may be done using methods well known in the art. In particular, quantification may be done through image analysis compared to the cells in a reference sample, for example in peripheral blood or bone marrow of a reference individual or multiple reference individuals, e.g. one or more healthy donor(s) where the cell sample, in particular the PBMC or bone-marrow cell sample is derived from a diseased donor. Quantification of cells is a standard diagnostic tool. Thresholds of cell subpopulations comprised in cell samples, in particular PBMCs and/or bone-marrow cells are well documented for healthy donors and diseased donors. Accordingly, based on differences in samples to be assessed using the means and methods of the present invention, the physiological-relevance can be determined. Documentation of cell subpopulations comprised in hematopoietic cells can be found, for example, in Hallek et al. (2008) Blood 111(12). Accordingly, quantification and further means and methods, for example determination of cell-cell interactions using microscopy, allow the determination whether a cell sample represents a physiological-relevant state.

In a preferred embodiment of the invention, the cells are analyzed in the form of monolayers. In particular, the monolayers are formed prior to the methods of the present invention and can allow, *inter alia,* imaging and/or microscopic analysis of cell populations, in particular PBMC populations and/or bone-marrow populations. Accordingly, monolayer as used herein implies a single layer of cells found predominantly within the same focal plane of the imaging device, *e.g.,* microscope or automated camera as is known in the art or described herein. The term single layer is used to mean that the cells within this layer form a culture that is predominantly 2-dimensional, *i.e.*, the culture is predominantly a layer of single cells. That is, within the culture, the majority of the cells are not found resting on or above other cells, and are not found in aggregates (e.g., consisting of groups of cells that extend above the layer of single cells by comprising cells that rest on or above other cells). Thus, the cell monolayer, in particular PBMC monolayer within the meaning of the invention preferably comprises a horizontal layer of cells, in particular PBMC cells having a thickness of the height of one single cell, in particular PBMC. Likewise, the bone-marrow cell monolayer within the meaning of the invention preferably comprises a horizontal layer of bone-marrow cells having a thickness of the height of one single bone-marrow cell. As used herein, the term monolayer does not exclude that within the culture vessel cell aggregates or multilayer constructs (*i.e.*, areas having cell cultures with a height of greater than one cell, in particular PBMC cell or one bone-marrow cell, respectively) or areas without cells may be found. Rather, the term is used to mean that the cultures of the invention will have the majority of their imageable or visible area (*e.g.*, by microscopic methods) consisting of a single layer of cells. This is most easily accomplished as providing a single layer of cells on a cell culture surface. However, as the skilled person will appreciate, other formats of cell samples may also be used in the methods of the present invention. That is, any cell representation may be used in the present invention as long as cell-cell interactions can be quantified.

In the case of non-adherent cells that are to be analyzed, for example PBMCs or bone marrow cells, it is known that such cells typically do not form strong contacts with cell-culture surfaces or strong cell-to-cell contacts. Therefore, the cell monolayers used in the present invention, in particular the PBMC monolayers used in various aspects of the present invention are not envisioned to be necessarily equivalent to monolayers of adherent cells as understood in the art, *i.e.*, comprising a layer of cells firmly attached, evenly spread, and covering the majority of the culture surface. Rather, in some embodiments, the cell monolayer, in particular the PBMC monolayer used in the invention may comprise cultures of high density comprising a majority of cells in direct contact with one or more other cells, but not necessarily adhered to the culture surface, or may comprise cultures of low density, wherein cells are within the monolayer but have no (direct physical) contact with any other cell in the culture. The cell monolayers used in certain aspects of the present invention may also comprise cultures of intermediate density, having discrete areas wherein cells are in contact with one or more cells and other areas where the cells exhibit no contact with other cells.

In some aspects, the invention relates to methods for determining whether a subject suffering from or predisposed to a disease will respond or is responsive to treatment with a therapeutic agent and/or a pharmaceutical composition comprising a compound for use in the treatment of a disease, in particular a hematologic malignancy and/or a malignancy of myeloid and/or lymphoid tissue of an individual, or disease affecting the immune system such as an inflammatory disease or autoimmune disease, wherein said therapeutic agent and/or pharmaceutical composition is selected from at least two or more test compounds, wherein each of the at least two or more test compounds is tested in a population of cells in an assay comprising the steps of (a) determination of the number of interactions between cells of a first distinguishable subpopulation and cells of a second distinguishable subpopulation comprised in the population of cells; (b) determination of the number of interactions in the population of cells by randomly assigning cells comprised in the population of cells to first and second distinguishable subpopulation of cells, wherein the absolute number of cells in the first and second distinguishable subpopulation of cells, respectively, are identical to the number of cells in the first and second distinguishable subpopulation of cells in (a); (c) determination of the propensity of cells to interact by dividing (a) through (b), wherein propensity increases with the resulting number; and (d) determination of the alteration of the propensity of cells to interact by comparing the propensity prior to addition of a therapeutic agent and subsequent to addition of a therapeutic agent and thereby determining whether the subject will respond or is responsive to treatment with the therapeutic agent, wherein the assay is repeated for each of the at least two or more compounds and wherein the compound reducing or enhancing the propensity of the cells to interact is selected for treatment / selected as component of the pharmaceutical composition.

The above method may also be used as a screening method of novel therapeutic agents/drugs. That is, in one embodiment of the present invention, a screening method is provided, wherein a therapeutic agent or pharmaceutical composition is screened by determining the alteration of the propensity of cells to interact in a population of cells due to addition of one or more test substance(s), wherein the population of cells comprises at least two distinguishable subpopulations of cells and wherein the method comprises (a) determination of the number of interactions between cells of a first distinguishable subpopulation and cells of a second distinguishable subpopulation; (b) determination of the number of interactions in the population of cells by randomly assigning cells comprised in the population of cells to first and second distinguishable subpopulation of cells, wherein the absolute number of cells in the first and second distinguishable subpopulation of cells are identical to the number of cells in the first and second distinguishable subpopulation of cells in (a); (c) determination of the propensity of cells to interact by dividing (a) through (b), wherein propensity increases with the resulting number; and (d) determination of the alteration of the propensity of cells to interact by comparing the propensity prior to addition of a test compound and subsequent to addition of a test compound and thereby determining whether the test compound altered/changed the propensity of cells to interact, wherein the assay is repeated for each of the at least two or more test compounds and wherein the test compound reducing or enhancing the propensity of the cells to interact is selected as a therapeutic agent/pharmaceutical composition. Preferably, the alteration/change of propensity is measured using the same population of cells but different fractions of the population of cells to determine the number of interactions prior and subsequent to addition of a test compound. This is particularly useful in case a monolayer of the cell sample is used and the monolayer is fixed prior to analysis.

Viability and/or cell-cell interactions of cells comprised in the monolayer can be determined and/or changes of viability and/or cell-cell interactions can be determined/assessed/tracked/verified using detectable labels/markers/dyes. Such labels/markers/dyes can be specific for one or more subpopulation(s) comprised in the monolayers of the invention. Where such specific labels/markers/dyes are used, they can be selected for cell types that play a role in various diseases and/or are known to have a biological function in a disease, in particular a hematologic malignancy and/or a malignancy of myeloid and/or lymphoid tissue.

Accordingly, in particular aspects, the invention relates to methods for determining whether a subject suffering from or predisposed to a disease will respond or is responsive to treatment with a therapeutic agent and/or a pharmaceutical composition comprising a compound for use in the treatment of a disease by determining the alteration of the propensity of cells to interact in a population of cells obtained from the subject, wherein the population of cells comprises at least two distinguishable subpopulations of cells and wherein the method comprises (a) determination of the number of interactions between cells of a first distinguishable subpopulation and cells of a second distinguishable subpopulation comprised in a cell sample obtained from the subject; (b) determination of the number of interactions in the cell sample by randomly assigning cells comprised in the cell sample to first and second distinguishable subpopulation of cells, wherein the absolute number of cells in the first and second distinguishable subpopulation of cells, respectively, are identical to the number of cells in the first and second distinguishable subpopulation of cells in (a); (c) determination of the propensity of cells to interact by dividing (a) through (b), wherein propensity increases with the resulting number; and (d) determination of the alteration of the propensity of cells to interact by comparing the propensity prior to addition of a therapeutic agent and subsequent to addition of a therapeutic agent and thereby determining whether the subject will respond or is responsive to treatment with the therapeutic agent. In an exemplary embodiment, first and second distinguishable subpopulation are CD3 and CD34 positive cells, respectively, or CD34 and CD3 positive cells, respectively. Preferably, the alteration/change of propensity is measured using the same cell sample but different fractions of the cell sample to determine the number of interactions prior and subsequent to addition of a test compound. This is particularly useful in case a monolayer of the cell sample is used and the monolayer is fixed prior to analysis.

The therapeutic agent used herein or the pharmaceutical composition of the invention comprises a compound selected from at least two or more test compounds.

Test compounds are not particularly limited as long as they are suitable for use as pharmaceutical. However, it is preferred that said test compounds are selected from compounds known to be effective in the treatment of a disease, in particular a hematologic malignancy and/or a malignancy of myeloid and/or lymphoid tissue, inflammatory and autoimmune diseases. Compounds known to be effective in the treatment of such diseases comprise chemical compounds and biological compounds, such as, for example, antibodies. Examples of compounds known to be effective in the treatment of such diseases include but are not limited to Alemtuzumab, Anagrelide, Arsenic trioxide, Asparaginase, ATRA, Azacitidine, Bendamustin, Blinatumomab, Bortezomib, Bosutinib, Brentuximab vedotin, Busulfan, Ceplene, Chlorambucil, Cladribine, Clofarabine, Cyclophosphamide, Cytarabine, Dasatinib, Daunorubicin, Decitabine, Denileukin diftitox, Dexamethasone, Doxorubicin, Duvelisib, EGCG = Epigallocatechin gallate, Etoposide, Filgrastim, Fludarabine, Gemtuzumab ozogamicin, histamine dihydrochloride, Homoharringtonine, Hydroxyurea, Ibrutinib, Idarubicin, Idelalisib, Ifosfamide, Imatinib, Interferon Alfa-2a, Recombinant, Interferon Alfa-2b, Recombinant, Intravenous Immunoglobulin, L-asparaginase, Lenalidomide, Masitinib, Melphalan, Mercaptopurine, Methotrexate, Midostaurin, Mitoxantrone, MK-3475 = Pembrolizumab, Nilotinib, Pegaspargase, Peginterferon alfa-2a, Plerixafor, Ponatinib, Prednisolone, Prednisone, R115777, RAD001 (Everolimus), Rituximab, Ruxolotinib, Selinexor (KPT-330), Sorafenib, Sunitinib, Thalidomide, Topotecan, Tretinoin, Vinblastine, Vincristine, Vorinostat, Zoledronate, ABL001, ABT-199 = Venetoclax, ABT-263 = Navitoclax, ABT-510, ABT-737, ABT-869 = Linifanib, AC220 = Quizartinib, AE-941 = Neovastat, AG-858, AGRO100, Aminopterin, Asparaginase Erwinia chrysanthemi, AT7519, AT9283, AVN-944, Bafetinib, Bectumomab, Bestatin, beta alethine, Bexarotene, BEZ235, BI 2536, Buparlisib (BKM120), Carfilzomib, Carmustine, Ceritinib, CGC-11047, CHIR-258, CHR-2797, CMC-544 = Inotuzumab ozogamicin, CMLVAX100, CNF1010, CP-4055, Crenolanib, Crizotinib, Ellagic Acid, Elsamitrucin, Epoetin Zeta, Epratuzumab, FAV-201, Favld, Flavopiridol, G4544, Galiximab, gallium maltolate, Gallium nitrate, Givinostat, GMX1777, GPI-0100, Grn163I, GTI 2040, IDM-4, Interferon alfacon-1, IPH 1101, ISS-1018, Ixabepilone, JQ1, Lestaurtinib, Mechlorethamine, MEDI4736, MGCD-0103, MLN-518 = Tandutinib, motexafin gadolinium, Natural alpha interferon, Nelarabine, Obatoclax, Obinutuzumab, OSI-461, Panobinostat, PF-114, PI-88, Pivaloyloxymethyl butyrate, Pixantrone, Pomalidomide, PPI-2458, Pralatrexate, Proleukin, PU-H71, Ranolazine, Rebastinib, Samarium (153sm) lexidronam, SGN-30, Skeletal targeted radiotherapy, Tacedinaline, Tamibarotene, Temsirolimus, Tioguanine, Troxacitabine, Vindesine, VNP 40101M, Volasertib, XL228, hydroxychloroquine (Plaquenil), leflunomide (Arava), methotrexate (Trexall), sulfasalazine (Azulfidine), minocycline (Minocin), abatacept (Orencia), rituximab (Rituxan), tocilizumab (Actemra), anakinra (Kineret), adalimumab (Humira), etanercept (Enbrel), infliximab (Remicade), certolizumab, pegol (Cimzia), golimumab (Simponi), tofacitinib (Xeljanz, Xeljanz XR), baricitinib, celecoxib (Celebrex), ibuprofen (prescription-strength), nabumetone (Relafen), naproxen sodium (Anaprox), naproxen (Naprosyn), piroxicam (Feldene), diclofenac (Voltaren, Diclofenac Sodium XR, Cataflam, Cambia), diflunisal, indomethacin (Indocin), ketoprofen (Orudis, Ketoprofen ER, Oruvail, Actron), etodolac (Lodine), fenoprofen (Nalfon), flurbiprofen, ketorolac (Toradol), meclofenamate, mefenamic acid (Ponstel), meloxicam (Mobic), oxaprozin (Daypro), sulindac (Clinoril), salsalate (Disalcid, Amigesic, Marthritic, Salflex, Mono-Gesic, Anaflex, Salsitab), tolmetin (Tolectin), betamethasone, prednisone (Deltasone, Sterapred, Liquid Pred), dexamethasone (Dexpak, Taperpak, Decadron, Hexadrol), cortisone, hydrocortisone (Cortef, A-Hydrocort), methylprednisolone (Medrol, Methacort, Depopred, Predacorten), prednisolone, cyclophosphamide (Cytoxan), cyclosporine (Gengraf, Neoral, Sandimmune), azathioprine (Azasan, Imuran), and hydroxychloroquine (Plaquenil).

The below table includes known drug - disease relations and cell markers that can be used to select the first and second distinguishable subgroup of cells. Moreover, the table indicates alterations/changes of the propensity of cells induced by the respective drug. Accordingly, such combinations can be directly implemented in the methods of the present invention.

Table II: Effect of immunomodulatory drugs on interaction propensity between cells defined by Marker 1 and Marker 2 that can be used to determine if a patient will respond to a particular treatment

| Target | Example Drug | Interaction | Function | Marker 1 | Marker 2 | Effect on interaction propensity |
|---|---|---|---|---|---|---|
| CTLA4 | Ipilimumab | Cytotoxic t-cells - Cancer cells | Drives cytotoxic immune function by blockinq CTLA4 | CD3/ CD8 | Cancer cell marker | Increase |
| CTLA4 | Tremelimumab | Cytotoxic t-cells - Cancer cells | Drives cytotoxic immune function by blockinq CTLA4 | CD3/ CD8 | Cancer cell marker | Increase |
| PD1 | Pembrolizumab | Cytotoxic t-cells - Cancer cells | Drives cytotoxic immune function by blocking PD1/PDL1 | CD3/ CD8 | Cancer cell marker | Increase |
| PD1 | Nivolumab | Cytotoxic t-cells - Cancer cells | Drives cytotoxic immune function by blocking PD1/PDL1 | CD3/ CD8 | Cancer cell marker | Increase |
| PDL1 | Atezolizumab | Cytotoxic t-cells - Cancer cells | Drives cytotoxic immune function by blockinq PD1/PDL1 | CD3/ CD8 | Cancer cell marker | Increase |
| PDL1 | MEDI4736 | Cytotoxic t-cells - Cancer cells | Drives cytotoxic immune function by blockinq PD1/PDL1 | CD3/ CD8 | Cancer cell marker | Increase |
| PDL1 | Avelumab | Cytotoxic t-cells - Cancer cells | Drives cytotoxic immune function by blocking PD1/PDL1 | CD3/ CD8 | Cancer cell marker | Increase |
| PD1 | PDR001 | Cytotoxic t-cells - Cancer cells | Drives cytotoxic immune function by blocking PD1/PDL1 | CD3/ CD8 | Cancer cell marker | Increase |
| CD137 | Urelumab | Cytotoxic t-cells - Cancer cells | Drives cytotoxic immune function by acrtivating CD137 | CD3/ CD8 | Cancer cell marker | Increase |
| OX40 | MEDI6469 | Cytotoxic t-cells - Cancer cells | Drives cytotoxic immune function by Blocking CD134 | CD3 | Cancer cell marker | Increase |
| GITR | TRX518 | Cytotoxic t-cells - Cancer cells | Drives cytotoxic immune function by blocking GITR | CD3 | Cancer cell marker | Increase |

(continued)

| Target | Example Drug | Interaction | Function | Marker 1 | Marker 2 | Effect on interaction propensity |
|---|---|---|---|---|---|---|
| CD27 | Varlilumab | Cytotoxic t-cells - Cancer cells | Antibody dependent and directed cytotoxic T cell-mediated tumor cell lysis by binding to CD27 on the cancer cell | CD3 | Cancer cell marker | Increase |
| CD40 | CP-870893 | Activated immune cell - cancer cell | Drives cytotoxic immune function by acrtivating CD40 | CD3 | Cancer cell marker | Increase |
| LAG3 | BMS-986016 | Cytotoxic t-cells - Cancer cells | Antibody dependent and directed cytotoxic T cell-mediated tumor cell lysis by binding to LAG3 on the cancer cell | CD3 (for one) | Cancer cell marker | Increase |
| B7-H3 | MGA271 | Cytotoxic t-cells - Cancer cells | Antibody dependent and directed cytotoxic T cell-mediated tumor cell lysis by binding to B7-H3- on the cancer cell | CD3 (for one) | Cancer cell marker | Increase |
| KIRs | Lirilumab | NK cells - Cancer cells | Induction of NK mediated tumor cell lysis by binding to KIR on the NK cell and inducing activation | CD56 | Cancer cell marker | Increase |
| NKG2D and NKG2A | IPH2201 | T-cell or NK-cell - Cancer cell | Induction of T-cell and NK cell medaited tumor cell lysis by binding to NKG2A and inducing acitvation | CD56 or CD3 | Cancer cell marker | Increase |
| IDO | INCB024360 | Cytotoxic t-cells - Cancer cells | Drives cytotoxic immune function by blockinq IDO | CD3/CD8 | Cancer cell marker | Increase |
| TGFβ | Galuniserti b | Cytotoxic t-cells - Cancer cells | Drives cytotoxic immune function by blocking TGFb signaling | CD3/CD8 | Cancer cell marker | Increase |
| CD39, CD73 | n/a | Cytotoxic t-cells - Cancer cells | Drives cytotoxic immune function by blocking inhibitory adenosine signaling | CD3/CD8 | Cancer cell marker | Increase |
| Glucoco rticoid receptor | Prednisone | Monocyte - monocyte | Antiinflammatory | CD14 | CD14 | Decrease |

The viability of cells comprised in the sample to be analyzed, in particular in the monolayers can be determined/assessed/verified using methods well-known in the art. That is, the skilled person is well-aware of methods how to determine/assess/verify the stadium of a cell, for example whether a cell is viable, live, dead or undergoing a process changing its stadium, for example dying as in apoptosis or necrosis. Accordingly, known markers/dyes that specifically recognize/label cells being in a particular stadium can be used in the methods of the invention. That includes dyes/labels that are selective for cells with non-intact membranes or dyes/labels selective for late-stage cell death or early apoptosis. For example, fixable live/dead green can be used (ThermoFisher, catalogue number L-23101), antibodies against cytochrome C, determining DNA turnover or cell proliferation through the use of dyes. Further means and methods how to determine/assess/verify viability of cells comprised in cell sample used in the present invention / the population of cells of the present invention, in particular in the form of a monolayer are known to the skilled person.

Determining/tracking/assessing/verifying changes of viability and/or cell-cell interactions of the two or more distinguishable subpopulation(s) comprised in the cell sample, in particular the monolayer, in particular PBMC monolayer or bone-marrow cell monolayer, can be done using methods well-known in the art. For example, using microscopy, changes can be determined/tracked/assessed/verified by optical perception. However, for high-throughput applications, it is preferred that an automated method is used, which determines/tracks/assesses/verifies changes of viability and/or cell-cell interactions of individual subpopulations comprised in the monolayers. Such a method comprises identifying subpopulations comprised in the cell sample, preferably the monolayer, e.g. by detectable labels. It can then be determined whether labeled/detected subpopulations show cell-cell interactions, wherein cell-cell interactions may include direct contacts via plasma membranes (as described above) or indirect contacts. Accordingly, a distance parameter, i.e. the threshold defined above, between labeled cells is introduced, which determines the total number of interactions, i.e. how many cell-cell interactions are observed between labeled cells. In this procedure, a labeled cell of a distinguishable subgroup may interact with one or more cells of the second distinguishable subgroup, each interaction being counted. The resulting number is compared to what would be expected by a random distribution function, i.e. by random cell-cell interactions. The interaction propensity can then be calculated using the methods of the invention, i.e. an interaction score, which determines whether interaction is random or directed. Following such a protocol before and after one or more test substance(s) are added to the cell sample of the invention, allows determining/tracking/assessing/verifying changes of cell-cell interactions due to the one or more test compound(s).

The present invention thus provides methods using physiologically relevant, multi-population cell samples, in particular primary hematopoietic samples in imaging studies to determine in a high-throughput manner: 1) the effects of chemotherapy / immunotherapy / immune suppressive therapy on *ex vivo* cell population diagnostic, or other, markers at a global level based on single-cell analysis, 2) the ability for this technique to provide predictive chemotherapy *ex vivo* in patient samples, 3) the ability for this technique to determine the effect of many stimuli or a stimulus (e.g. drugs) on the immune function, and 4) for the integration of many patient data sets over time to determine patterns in treatment assessments. In principle, any cell sample may be used in the methods of the present invention such as mononuclear cells from blood, bone marrow, pleural effusion, spleen homogenates, lymph tissue homogenates, skin homogenates. However, it is preferred that mononuclear cells are used. As the skilled person is aware, mononuclear cells samples as used in the methods of the invention comprise, *inter alia,* PBMCs and bone-marrow cells, as well as others. Accordingly, the cell sample, preferably the monolayer of primary mononuclear cells as provided herein and as used in the methods of the invention may comprise PBMCs and/or bone-marrow cells. That is, while the means and methods provided herein are described for cells in general or PBMCs, the skilled person understands that identical means and methods are provided for bone-marrow cells or further cells. Accordingly, provided herein are methods using bone-marrow cells, methods for determining whether a bone-marrow cell donor suffers from a disease or is predisposed to suffer from a disease, methods for diagnosing a disease or predisposition to a disease in a bone-marrow donor and methods for determining whether a subject suffering from or predisposed to a disease will respond or is responsive to treatment with a therapeutic agent comprising the use of bone-marrow cells. Furthermore, methods for drug screening and other methods provided herein are also disclosed for other hematopoietic cells, e.g. bone-marrow cells.

In this regard, bone marrow is the flexible tissue in the interior of bones. In humans, red blood cells are produced by cores of bone marrow in the heads of long bones in a process known as hematopoiesis. Bone marrow transplants can be conducted to treat severe diseases of the bone marrow, including certain forms of cancer such as leukemia. Additionally, bone marrow stem cells have been successfully transformed into functional neural cells and can also be used to treat illnesses such as inflammatory bowel disease. Accordingly, bone-marrow cells represent a valuable target in the treatment of various diseases, for example cancerous diseases or inflammatory diseases such as inflammatory bowel disease. As such, the methods provided herein using bone-marrow samples obtained from a donor are highly useful in the assessment/determination whether a donor suffers from such a disease or is predisposed to suffer from a disease. In addition, the methods provided herein using bone-marrow cells provide various advantages in high-throughput drug screening and the like.

The dogma of requiring adherent cells (macrophages, HeLa, etc.) to form a stainable and imageable monolayer has been overcome by the provision of monolayers in WO 2016/046346. Prior to the monolayers as described therein, research groups have been unable to implement image-based single cell screening techniques in primary patient samples for high-throughput determination of chemotherapy-induced molecular (biomarker) changes, cancerous blast viability assessments and cell-cell contacts, in particular where the disease state is represented or reflected in non-adherent cells, e.g., in blood-based diseases or conditions such as lymphomas and leukemias. To solve this problem, the inventors of WO 2016/046346 have provided means and methods as well as a methodology and image-analysis pipeline, referenced herein as "pharmacoscopy", which allows the visualization of adherent and non-adherent cells in a single image, typically requiring only 1/10th of the material needed per perturbation as compared to methods known in the art, and maximizing throughput and speed. Pharmacoscopy can provide the same information gathered by known methods, e.g., flow cytometry, but provides additional advantageous information such as measurement of subcellular phenotypes (protein localization/colocalization) and cellular microenvironment / neighbor relationship. Moreover, the described methods of WO 2016/046346 require fewer cells and therefore less patient material, less liquid volume, and nearly no human intervention; pharmacoscopy thereby greatly increases the number of molecular perturbations which can be tested in parallel and yields more detailed assessments. Moreover, without the need to sort diseased cells from the inherent healthy populations, pharmacoscopy can track drug mediated biomarker changes while controlling, in parallel, the off-target drug effects. These important controls are done by tracking the cell interaction patterns of the healthy cells from the same donor, present in the same well, and in the same imaging field, to the cell interaction pattern and biomarker analysis of the targeted-cell populations. The methods of the present invention use the methodology of WO 2016/046346, but comprise a further surprising and unexpected advantage. In particular, it is now possible to analyze cell samples undergoing changes in cell population, e.g. where cells are killed during the experiment, and cell samples having a high cell density in a more reliable manner.

Using the methods of the present invention, the analysis of drug-induced sub-cellular and single-cell biomarker changes, within patient blood samples, can predict clinical therapy outcomes tailored to individual patients. The standardization, perfection, and availability of this technology to basic research as well as medical professionals and clinics is an advantage for, inter alia, personalized medicine, predictive pharmacology as well as drug screening and therapeutic evaluation.

Accordingly, and in contrast to the prior art, the methods as provided herein can be employed in an even wider variety of applications than methods described in WO 2016/046346, e.g. personalized medicine, drug screening programs, general drug screening, personalized drug screening, assessment of drug response, assessment of the immunological properties of a drug, evaluation of treatment, verification of treatment efficacy, prediction of treatment response, population (drug) responses and the like, of cell samples having a high cell density and/or cell samples undergoing changes in the number of cells comprised in the cell sample. The means and methods provided herein, thus, allow also drug screenings and drug discoveries as well as personalized (i.e., subject/patient/individual related) drug discoveries or drug screenings. For example, in general drug discoveries and/or drug screenings as provided herein, cells, in particular PBMCs or bone-marrow cells, of healthy versus diseased patients may be used and compared. Also, pooled cell samples, in particular PBMC samples or bone-marrow samples, may be employed as starting material for the inventive monolayers in the means and methods provided herein. In personalized drug discovery, preferably, individual PBMC samples of subjects/individuals/patients are employed as starting material for the inventive PBMC monolayers to be employed in accordance with the invention.

Using the methods of the invention large numbers of perturbations can be efficiently and quickly investigated using the large numbers of monolayers that may be derived from a single sample obtained from a patient, in particular a PBMC sample or bone-marrow sample. Typically, the effect of at least 1000, at least 4000, at least 8000, at least 12000, at least 16000, at least 20000, at least 24000, at least 50000, at least 75000, or up to 90000 compounds or more can be investigated in the multiple monolayers obtained from such a single sample. In certain embodiments, the monolayers provided herein can be imaged and analyzed using multiple channels simultaneously of high content data. The number of channels of data available is dependent only on the particular imaging software and available staining methodologies, which field rapidly advances. Currently available methodologies allow the simultaneous imaging, processing and analysis of at least two channels, and more typically, 4, 5 or 8 channels of high-content data.

Peripheral blood mononuclear cells (PBMCs) are blood cells having a round nucleus (monocytes; as opposed to a lobed nucleus). PBMCs comprise lymphocytes (B-cells, T-cells (CD4 or CD8 positive), and NK cells), monocytes (dendritic cell and macrophage precursor), macrophages, and dendritic cells. These blood cells are a critical component in the immune system to fight infection and adapt to intruders. In context of some embodiments of the present invention, it is preferred to use ficoll density gradient purified PBMCs, preferably human PBMCs, for creation of the PBMC monolayer of the invention or the cell-culture device comprising the PBMC monolayer or for use in the methods provided in some aspects of the present invention. The present invention can be used with any mononuclear cells. In a preferred embodiment, the invention can determine, but is not limited to determining, the interaction score of the cells within the following groups of cells, and cells within the lineage of the cells, including terminal cell states: Hematopoietic stem cells (including, but not limited to, common lymphoid progenitor, common myeloid progenitor, and their maturation lineage and terminal states including pro-B-cell, B-cell, double negative t-cells, positive T-cell, plasma-B-cell, NK-cells, monocytes (macrophage, dendritic cells)). These can be found, but not limited to, within peripheral blood, bone marrow (flat bone localized), cord blood, spleen, thymus, lymph tissue, and any fluid buildup result of a disease such as pleural fluid. Cells may be in any healthy or diseased state.

PBMCs cells for use according to the methods described herein can be isolated from whole blood using any suitable method known in the art or described herein. For example, the protocol described by Panda et al. may be used (Panda, S. and Ravindran, B. (2013). Isolation of Human PBMCs. Bio-protocol 3(3): e323). Preferably, density gradient centrifugation is used for isolation. Such density gradient centrifugation separates whole blood into components separated by layers, e.g., a top layer of plasma, followed by a layer of PBMCs and a bottom fraction of polymorphonuclear cells (such as neutrophils and eosinophils) and erythrocytes. The polymorphonuclear cells can be further isolated by lysing the red blood cells, i.e. non-nucleated cells. Common density gradients useful for such centrifugation include, but are not limited to, Ficoll (a hydrophilic polysaccharide, e.g., Ficoll®-Paque (GE Healthcare, Upsalla, Sweden) and SepMate™ (StemCell Technologies, Inc., Köln, Germany).

Bone-marrow cells for use according to the methods described herein can be isolated from bone marrow using any suitable method known in the art. In particular, magnetic beads can be used to separate bone-marrow cells from other components of such samples. For example, MACS cell separation reagents may be used (Miltenyi Biotec, Bergisch Gladbach, Germany).

As is known in the art, such isolated cultures may contain a small percentage of one or more populations of another cell type, e.g., non-nucleated cells such as red blood cells. The PBMCs may be further isolated and/or purified from such other cell populations as is known in the art and/or as described herein; for example, methods of lysing red blood cells is commonly use to remove such cells from the isolated PBMCs. However, the methods of the invention are not reliant on further purification methods, and the isolated PBMCs isolated herein may be directly used. Accordingly, the methods disclosed herein may or may not comprise lysing of red blood cells from within the sample of isolated PBMCs. However, where present, it is believed that the presence of non-nucleated cells, e.g., red blood cells, being generally smaller than PBMCs, settle on the culture surface below and between the PBMCs, and potentially interfere with the formation of a monolayer suitable for imaging. Therefore it is preferred that the concentration of non-nucleated cells, e.g., red blood cells, relative to PMBCs is between about 500 to 1, more preferably about 250 to 1, most preferably about 100 to 1, with the preferential concentration as low as possible. That is, it is most preferred that the isolated PBMC sample according to the methods disclosed herein contains less than about 100 non-nucleated cells, e.g. red blood cells, per PBMC.

In some embodiments of the methods of the invention and/or in order to provide the monolayers used in the invention, cells, in particular PBMCs, are incubated subsequently to isolation at a density of about 100 cells per $mm^2$ growth area to about 30000 cells per $mm^2$ growth area. Preferably, the cells, in particular PBMCs, are incubated at a density of about 500 cells per $mm^2$ growth area to about 20000 cells per $mm^2$ growth area, about 1000 cells per $mm^2$ growth area to about 10000 cells per $mm^2$ growth area, about 1000 cells per $mm^2$ growth area to about 5000 cells per $mm^2$ growth area, or about 1000 cells per $mm^2$ growth area to about 3000 cells per $mm^2$ growth area. Most preferably the cells, in particular PBMCs, are incubated at a density of about 2000 cells per $mm^2$ growth area. The term "about" shall have the meaning of within 10%, more preferably within 5%, of a given value or range. Accordingly, the cells, in particular PBMCs, are, in some embodiments, incubated using methods of the invention to have in the culture device a density of about 100, i.e. from 90 to 110, cells per $mm^2$ growth area to about 30000, i.e. 27000 to 33000, cells per $mm^2$ growth area. More preferably, the cells, in particular PBMCs, are incubated at a density of about 500, i.e. 450 to 550, cells per $mm^2$ growth area to about 20000, i.e. 18000 to 22000, cells per $mm^2$ growth area, about 1000, i.e. 900 to 1100, cells per $mm^2$ growth area to about 10000, i.e. 9000 to 11000, cells per $mm^2$ growth area, about 1000, i.e. 900 to 1100, cells per $mm^2$ growth area to about 5000, i.e. 4500 to 5500, cells per $mm^2$ growth area, or about 1000, i.e. 900 to 1100, cells per $mm^2$ growth area to about 3000, i.e. 2700 to 3300, cells per $mm^2$ growth area. Most preferably the cells, in particular PBMCs, are incubated at a density of about 2000, i.e. 1800 to 2200, cells per $mm^2$ growth area.

The number of cells, in particular PBMCs, can be determined using standard methods known in the art. In particular, the number of PBMCs can be determined by cell counting using a hemocytometer or the method described by Chan et al. (Chan et al. (2013) J. Immunol. Methods 388 (1-2), 25-32). The number of bone-marrow cells can also be determined using methods well known in the art. In particular, bone-marrow cells can be determined using cell counting. Other cells may also be counted using methods well-known in the art.

Incubation is carried out in a culture medium. A person skilled in the art is well aware of suitable methods to maintain viability of cells, in particular PBMCs or bone-marrow cells. However, the culture medium to be used in the methods of the invention is not particularly limited. In this regard, medium stands for liquids with nutrients and substances necessary for cultivation of cells. Liquid culture media for culturing eucaryotic cells are known to the person skilled in the art (e.g., DMEM, RPMI 1640, etc). Suitable media may be selected depending on the type of cells to be cultured. For example, PBMCs or bone-marrow cells may be cultivated in RPMI 1640 10% FCS. Any suitable media may be chosen, however, media components should be selected that are known to not artificially influence PBMC response and/or bone-marrow cell response. Supplements describe substances to be added to culture media in order to induce or modify cell function (e.g. cytokines, growth and differentiation factors, mitogens, serum). Supplements are known to the person of skill in the art. One example of a serum commonly used with eukaryotic cells is fetal calf serum. The culture media may further be supplemented with antibiotics, such as penicillin, streptomycin, ciprofloxacin etc. In one embodiment, test substances and/or stimulatory agents may be added to living cell material in each individual unit separately. Test substances may be pharmaceutical drugs or drug components.

Stimulators may comprise any of the substances which support maintenance, growth or differentiation of cells. In a particular embodiment, stimulators are substances which act on immune cells, e.g. by activation of immune cells. Stimulators for activation of immune cells are known from the prior art. Such agents may be polypeptides, peptides or antibodies and other stimulators. For example, OKT-3, interferon-alpha, interferon-beta and interferon-gamma, oligoCPGs, mitogens (e.g. PWM, PHA, LPS), etc. Test substances and stimulators may be injected into the cell culture medium. Preferably, PBMCs are cultured in RPMI supplemented with FBS/FCS at 10% (preferably but not necessarily having low endotoxin raitings to minimize activation). PBMC cultures may furthermore comprise human serum from the PBMC donor.

The term "growth area" as used within the meaning of the invention refers to the surface within a culture device upon which cells rest. The "density" as used within the meaning of the invention is the quantity of cells per unit area of the surface within the device upon which the cells rest. The culture device may be produced of any material compatible with cell culture, in particular, non-cytotoxic cell culture tested material. Examples for the material are plastic materials, e.g., thermoplastic or duroplastic materials. Examples of suitable plastics are polyethylene, polypropylene, polysulfone, polycarbonate, polyetherethylketone (PEEK) or polytetrafluorethylene (PTFE). In particular, the device is suitable for the culture and/or maintenance of PBMCs. Typical culture devices known in the art and of use in the invention include culture flasks, dishes, plates, and multi-well plates. Of particular use are multi-well plates, which provide the ability to separately maintain multiple cultures, *e.g.,* for multiple perturbations, with minimal material requirements, *e.g.,* minimal media requirements. Preferred culture devices include 96 well plates, 384 well plates and 1536 well plates. As known in the art in connection with imaging analysis of cultures, in particular, fluorescence imaging, it is particularly preferred to use black wall plates specifically designed for imaging that reduce background fluorescence / background optical interference with minimal light scatter and reduced crosstalk. The culture device may be sterilized. In a most preferred embodiment, a multiwell imaging plate is used, the plate including multiple wells, wherein at least some of the wells comprise a first chamber, the first chamber being formed by one or more first sidewalls and a bottom wall; a second chamber, the second chamber being formed by one or more second sidewalls and including an opening for introducing liquids, wherein the second chamber is arranged on top of the first chamber; an intermediate floor provided between the first chamber and the second chamber which forms a disturbance blocking structure; wherein the intermediate floor is provided with at least one through hole that provides a liquid connection between the first and second chambers; wherein the through hole is configured for a tip of a pipette being inserted through the second chamber into the first chamber through said through hole.

The device is in particular of use in automated imaging systems and analysis. Thus, it is preferred that the device/culture device is suitable for use in such systems. In a non-limiting example, the culture device may be translucent. Culture dishes and plates of use for imaging, *e.g.,* fluorescent imaging, are well known in the art and are commercially available. A non-limiting example of a commercially available culture plate for use in the practice of the invention is Corning® 384-well, tissue-culture treated black lid, clear bottom plates (Corning Inc., Massachusetts, USA) or Corning® 384 Well Flat Clear Bottom Black Polystyrene TC-Treated Microplates (Product #3712). Another example is the Perkin Elmer Cellcarrier®.

The population of cells of the invention, preferably in the form of monolayers may be imaged according to any methods known in the art and/or described herein and the methods provided herein may use any imaging technique known in the art. The particular imaging method is not critical and may be decided according to the knowledge of the person of skill in the art. The imaging may or may not require the use of a dye or stain, may comprise imaging of both stained and non-stained components and/or may comprise imaging under conditions wherein the stain is or is not visible (*e.g.,* imaging in bright-field (wherein a fluorescent stain would not be visible) and under uv-lighting (wherein a fluorescent stain would be visible), or combinations thereof. Imaging under bright-field conditions is well known and routine used in the art, and may be performed according to standard methods and/or as described herein. Additionally or alternatively, any other label-free imaging may be used in accordance with the invention. Such label-free methods are known and include, *e.g.,* PhaseFocus imaging (Phase Focus Ltd, Sheffield, UK).

The practice of the invention may also comprise the addition of a detectable label to the population of cells, preferably the monolayer, in particular the PBMC monolayer (either in connection with label-free methods or independently), which label may be detected using microscopic methods. The detectable labels may label discrete cellular structures, components or proteins as known in the art. The label may also be attached to antibodies to specifically label and allow the detection of the antibody antigen. In a preferred embodiment, the detectable label allows visualization of the label under visible or ultra-violet light. Thus the detectable label may be fluorescent. A multitude of visual labels are known in the art and are suitable for the invention. The labels may be detectable without further action, or may only become detectable after performance of a secondary step, *e.g.,* addition of a substrate, exposure to enzymatic reactions, or exposure to specific light wavelengths.

Cellular subpopulations (target cells), i.e. distinguishable subpopulations as used herein, in particular PBMC subpopulations or bone-marrow cell subpopulations may be identified by detectable labels via expression of one or more markers on the surface of the target cell or inside of the cell. Alternatively or additionally, subpopulations may be defined by the lack of expression of one or more markers on the surface of the target cell or inside the target cell. It may be desirable to test for expression or lack of expression of one or more markers (e.g., two markers, three markers, four markers, etc.) to provide further assurance that a cell expressing or not expressing a marker is in fact a target cell, *e.g.,* a member of desired subpopulation of cells. For example, a "cocktail" of antibodies to different markers may be each coupled (whether directly or indirectly) to the same label or to different labels. As an example, a cocktail of antibodies to different markers may each contain a binding motif that binds the same label (e.g., each may contain an Fc of the same species that is recognized by the same secondary antibody, or each may be biotinylated and specifically bound by the same avidin-coupled label). Optionally, two or more different antibodies or cocktails of antibodies may be utilized. Preferably the cells are stained using at least two labels that can be distinguished from one another, thereby permitting identification of cells that express at least two different markers of the target cell types. Cells may also be stained using at least three, four, five, or more different labels that can be distinguished from one another, thereby permitting detection of cells that express greater numbers of markers of the target cell type. Optionally, a cell may be identified as a cell of the target type if it expresses a preselected number of markers or certain preselected combinations of markers or a cell may be identified as a cell of the target type if it does not express a preselected marker. Additionally, it is not necessary that the marker(s) of the target cell type be unique to the target cells, as long as they permit distinction of the target cells from other cells in the population. In the case of PBMCs, major components of PBMC cell populations are represented by CD11C for dendritic cells, CD14 for macrophages, CD3 (CD4 or CD8 with CD3) for T-cells and CD19 for B-cells. While the foregoing markers overlap on subsets of these major classes of PBMCs, staining with these markers for identifying subpopulations of PBMCs is widely accepted in the field. Further markers suitable for use in methods of the present invention may be found in the CD marker handbook (Becton, Dickinson and Co. 2010, CA, USA). Major cell subpopulations comprised in bone-marrow cells are neutrophilic metamyelocytes, neutrophilic myelocytes, segmented neutrophils, normoblasts and lymphocytes.

It is preferred to use antibodies conjugated to detectable labels in the practice of the invention. Such antibodies allow the targeting of discrete cellular structures and, thus, cocktails of such antibodies (each bearing a different label) may be used to simultaneously visualize multiple targets/cellular structures/cellular components. Care must be taken during staining to avoid monolayer disruption. As the skilled person appreciates, this is particularly problematic with the use of antibody-based labels, as their use normally requires one or more wash-steps to eliminate unbound label that would interfere with accurate visualization, *i.e.,* would result in non-specific staining and/or assay "noise". Accordingly, the invention encompasses methods for the staining of cell monolayers with a detectable label, in particular, an antibody-based label, which minimizes or eliminates washing requirements subsequent to staining. The methods of the invention may comprise adding the detectable label(s) at concentrations that avoid generation of noise signal in the absence of washing, which can be determined by methods well known in the art and/or described herein.

Thus, the invention encompasses the use of labeled antibodies at concentrations above or below that recommended by the antibody manufacturers.

For some exemplary cell types, cells may only be considered positive for a given marker if that marker exhibits a characteristic localization or pattern within the cell. For instance, a cell may be considered "positive" if a cytoskeletal marker is present in the cytoskeleton and "negative" if there is some diffuse cytoplasmic staining. In such a case, cells may be cultured under suitable conditions (e.g., as adherent cultures) to establish the characteristic localization or pattern within the cell. Suitable culture conditions and time for cytoskeleton assembly (or other processes to establish subcellular organization) that may be necessary for robust detection of a given marker are readily determined by those of ordinary skill in the art. Additionally, markers may readily be chosen which decrease or eliminate the need for adherent culture as a precondition to robust staining.

Dyes useful in labeling proteins are known in the art. In general, a dye is a molecule, compound, or substance that can provide an optically detectable signal, such as a colorimetric, luminescent, bioluminescent, chemiluminescent, phosphorescent, or fluorescent signal. In a preferred embodiment of the invention, the dye is a fluorescent dye. Non-limiting examples of dyes, some of which are commercially available, include CF dyes (Biotium, Inc.), Alexa Fluor dyes (Invitrogen), DyLight dyes (Thermo Fisher), Cy dyes (GE Healthscience), IRDyes (Li-Cor Biosciences, Inc.), and HiLyte dyes (Anaspec, Inc.). In some embodiments, the excitation and/or emission wavelengths of the dye are between 350 nm to 900 nm, or between 400 nm to 700 nm, or between 450-650 nm.

For example, staining may comprise using multiple detectable labels, e.g. antibodies, self-antibodies or patient serum. A stain may be observable under visible light and under ultraviolet light. A stain may comprise an antibody directly or indirectly coupled to a colored reagent or an enzyme capable of producing a colored reagent. When antibodies are used as a component of a stain, a marker can be directly or indirectly coupled to the antibody. Examples of indirect coupling include avidin/biotin coupling, coupling via a secondary antibody, and combinations thereof. For example, cells may be stained with a primary antibody that binds a target-specific antigen, and a secondary antibody that binds the primary antibody or a molecule coupled to the primary antibody can be coupled to a detectable marker. Use of indirect coupling can improve signal to noise ratio, for example by reducing background binding and/or providing signal amplification.

The stain may also comprise a primary or secondary antibody directly or indirectly coupled (as explained above) to a fluorescent label. The fluorescent label may be selected from the group consisting of: Alexa Fluor 350, Alexa Fluor 405, Alexa Fluor 430, Alexa Fluor 488, Alexa Fluor 514, Alexa Fluor 532, Alexa Fluor 546, Alexa Fluor 555, Alexa Fluor 568, Alexa Fluor 594, Alexa Fluor 610, Alexa Fluor 633, Alexa Fluor 635, Alexa Fluor 647, Alexa Fluor 660, Alexa Fluor 680, Alexa Fluor 700, Alexa Fluor 750 and Alexa Fluor 790, fluoroscein isothiocyanate (FITC), Texas Red, SYBR Green, DyLight Fluors, green fluorescent protein (GFP), TRIT (tetramethyl rhodamine isothiol), NBD (7-nitrobenz-2-oxa-1,3-diazole), Texas Red dye, phthalic acid, terephthalic acid, isophthalic acid, cresyl fast violet, cresyl blue violet, brilliant cresyl blue, para-aminobenzoic acid, erythrosine, biotin, digoxigenin, 5-carboxy-4',5'-dichloro-2',7'-dimethoxy fluorescein, TET (6-carboxy-2',4,7,7'-tetrachlorofluorescein), HEX (6-carboxy-2',4,4',5',7,7'-hexachlorofluorescein), Joe (6-carboxy-4',5'-dichloro-2',7'-dimethoxyfluorescein) 5-carboxy-2',4',5',7'-tetrachlorofluorescein, 5-carboxyfluorescein, 5-carboxy rhodamine, Tamra (tetramethylrhodamine), 6-carboxyrhodamine, Rox (carboxy-X-rhodamine), R6G (Rhodamine 6G), phthalocyanines, azomethines, cyanines (e.g. Cy3, Cy3.5, Cy5), xanthines, succinylfluoresceins, N,N-diethyl-4-(5'-azobenzotriazolyl)-phenylamine, aminoacridine, and quantum dots.

Further exemplary embodiments of the present method utilize antibodies directly or indirectly coupled to a fluorescent molecule, such as ethidium bromide, SYBR Green, fluorescein isothiocyanate (FITC), DyLight Fluors, green fluorescent protein (GFP), TRIT (tetramethyl rhodamine isothiol), NBD (7-nitrobenz-2-oxa-1,3-diazole), Texas Red dye, phthalic acid, terephthalic acid, isophthalic acid, cresyl fast violet, cresyl blue violet, brilliant cresyl blue, para-aminobenzoic acid, erythrosine, biotin, digoxigenin, 5-carboxy-4',5'-dichloro-2',7'-dimethoxy fluorescein, TET (6-carboxy-2',4,7,7'-tetrachlorofluorescein), HEX (6-carboxy-2',4,4',5',7,7'-hexachlorofluorescein), Joe (6-carboxy-4',5'-dichloro-2',7'-dimethoxyfluorescein) 5-carboxy-2',4',5',7'-tetrachlorofluorescein, 5-carboxyfluorescein, 5-carboxy rhodamine, Tamra (tetramethylrhodamine), 6-carboxyrhodamine, Rox (carboxy-X-rhodamine), R6G (Rhodamine 6G), phthalocyanines, azomethines, cyanines (e.g. Cy3, Cy3.5, Cy5), xanthines, succinylfluoresceins, N,N-diethyl-4-(5'-azobenzotriazolyl)-phenylamine and aminoacridine. Other exemplary fluorescent molecules include quantum dots, which are described in the patent literature [see, for example, U.S. Pat. Nos. 6,207,299, 6,322,901, 6,576,291, 6,649,138 (surface modification methods in which mixed hydrophobic/hydrophilic polymer transfer agents are bound to the surface of the quantum dots), U.S. Pat. Nos. 6,682,596, 6,815,064 (for alloyed or mixed shells), each of which patents is incorporated by reference herein)], and in the technical literature [such as "Alternative Routes toward High Quality CdSe Nanocrystals," (Qu et al., Nano Lett., 1(6):333-337 (2001)]. Quantum dots having various surface chemistries and fluorescence characteristics are commercially available from Invitrogen Corporation, Eugene, Oreg., Evident Technologies (Troy, N.Y.), and Quantum Dot Corporation (Hayward, Calif.), amongst others. Quantum dot" also includes alloyed quantum dots, such as ZnSSe, ZnSeTe, ZnSTe, CdSSe, CdSeTe, ScSTe, HgSSe, HgSeTe, HgSTe, ZnCdS, ZnCdSe, ZnCdTe, ZnHgS, ZnHgSe, ZnHgTe, CdHgS, CdHgSe, CdHgTe, ZnCdSSe, ZnHgSSe, ZnCdSeTe, ZnHgSeTe, CdHgSSe, CdHgSeTe, InGaAs, GaAlAs, and InGaN. Alloyed quantum dots and methods for making the same are disclosed, for example, in US Application Publication No. 2005/0012182 and PCT Publication WO 2005/001889.

Subsequent to labeling the population of cells of the invention, preferably in the form of a monolayer, the method may further comprise detecting the signal of the detectable label. Depending on the kind of signal emitted by the detectable label, the detection method may be appropriately adapted. It is preferred to use a detection method suitable for detecting fluorescent light emitting labels. The detection method may also be automated according to standard methods known in the art. For example, various computational methods exist that enable a person skilled in the art to analyze and interpret the microscopy images of cells or to establish automated protocols for their analysis. For primary image analysis, including the correction for illumination bias in microscopy images, the identification of individual cells from microscopy images and the measurement of marker intensities and textures as well as nuclear and cellular size and shape and position parameters, the opensource software CellProfiler (e.g. version 2.1.1) can be used. Identification of marker-positive cells (such as CD34+ progenitor cells or viability dye positive cells) can be performed by machine learning using the opensource software CellProfiler Analyst (e.g. version 2.0) and double- or triple-positive cells can be identified by a sequential gating strategy. Plate-overviews for further analysis and hit selection can be created using CellProfiler Analyst as well.

The cellHTS package in Bioconductor (e.g. version 2.14), or Pipeline Pilot (e.g. version 9.0; Accelrys), can both be used for the data analysis subsequent to the primary image analysis, including plate-effect normalization, control-based normalization, and hit selection.

Commercial automated microscopy systems may also be used in the practice of the invention, *e.g.,* PerkinElmer Operetta automated microscope (PerkinElmer Technologies GmbH & Co. KG, Walluf, Germany), which systems may include corresponding image analysis software, *e.g.,* PerkinElmer's Harmony software (e.g. version 3.1.1). Such automated and/or commercial systems can be used to perform primary image analysis, positive cell selection and hit selection from microscopic images according to the methods of the invention.

Subsequent to this primary analysis, the methods of the present invention are performed for determining the propensity of cells to interact in a population of cells comprising at least two distinguishable subpopulations of cells or for diagnosing a disease or predisposition to a disease of a cell donor or for screening for novel therapeutic agents, wherein the method comprises the determination of the propensity of cells to interact in a cell sample obtained from the donor, wherein the cell sample of the donor comprises at least two distinguishable subpopulations of cells or for determining whether a subject suffering from or predisposed to a disease will respond or is responsive to treatment with a therapeutic agent comprising the determination of the alteration of interaction between cells in a cell sample obtained from the subject, wherein the cell sample of the subject comprises at least two distinguishable subpopulations of cells.

The methods may further include isolating cells, in particular PBMCs or bone-marrow cells, from a subject previously or currently treated for an immune mediated disease, stimulating the PBMCs or bone-marrow cells, identifying subpopulations of PBMCs or bone-marrow cells, comparing cell interaction propensity data from the PBMC subpopulations or bone-marrow cell subpopulations to a subject response to the therapeutic and selecting a signature cell interaction profile related to a positive response to the therapeutic, thereby monitoring the course of therapy.

Immune mediated diseases can be divided into several categories including immunodeficiency diseases, autoimmune diseases and hypersensitivity diseases. Immunodeficiency diseases occur when part of the immune system is not functioning properly. Autoimmune diseases are the result of the immune system attacking the body instead of pathogens. Hypersensitivity diseases occur when the immune system over reacts and results in damage to the body.

The disease to be diagnosed using the methods of the invention is not particularly limited as long as it can be diagnosed using the cells comprised in the population of cells, preferably comprised in the monolayer, in particular as long as it can be diagnosed using PBMCs or bone-marrow cells, respectively, i.e. a subject having the disease to be diagnosed shows (an) altered cellular interaction pattern(s) of distinguishable subpopulations of cells comprised in the cell sample, e.g. PBMCs or bone-marrow cells that can be associated with the disease from those expected in a healthy donor. For example, diseases that can be diagnosed using the means and methods provided in the present invention include but are not limited to hematologic malignancies and/or a malignancy of myeloid and/or lymphoid tissue or immune mediated diseases. Diseases that are in particular diagnosable and/or predictable by the means and methods provided herein, in particular the PBMC monolayer or bone-marrow cell monolayer include, but are not limited to, myeloproliferative disorders (or general blood cancers), inflammatory disorders, latent virus infections, cellular growth disorders, cellular chemotaxis disorders, metabolic disorders, autoimmune disorders (e.g., staining with self ligand or patient serum for clonal antibodies or self antigen recognition). Moreover, the methods of the invention can be used to diagnose leukemia (chronic and acute), lymphoma (mature B-cell, mature T- and NK cell, Hodgkin's lymphoma), HIV, gout, shock and the like. That is, the means and methods of the present invention can be used to diagnose or determine whether a subject will respond / is responsive to treatment of the following diseases of the following ICD-10 codes (not limited thereto) A00-B99 - certain infectious and parasitic diseases; C00 - C97 malignant neoplasms; D70-77 - other diseases of the blood forming system; D80-89 - certain disorders involving the immune mechanism, not classified elsewhere; D82 - Immunodeficiency associated with other major defects; D83 - Common variable immunodeficiency; D84 - Other immunodeficiency; G35-37 - Diseases of the central nervous system; I00 - I03 - acute rheumatic fever; I05-I09 - Chronic rheumatic heart disease; I01 - Rheumatic fever with heart involvement; I06 - Rheumatic aortic valve diseases; I09 - Rheumatic myocarditis; I70 - Atherosclerosis; K50 - Crohn's disease; K51 - Colitis; K52 - other noninfective gastroenteritis and colitis; M00 - M19 Athropathies; M05 - Seropositive rheumatoid arthritis; M06 - Other rheumatoid arthritis; M10 - Gout; M11 - Other crystal athropathies; M35 - sicca syndrome; M32 - Systemic lupus erythematosus; N70-77 - inflammatory diseases of female pelvic organs; P35-39 - infections specific to the perinatal period; P50 - P61 - hemorrhagic and hematological disorders of the fetus and newborn; Z22 - Carrier of infectious disease; Z23 - Need for immunization against single bacterial diseases; and/or Z24 - Need for immunization against certain single viral diseases.

"Treatment" or "treating" refers to both therapeutic treatment and prophylactic or preventative measures, wherein the object is to prevent, ameliorate or slow down (lessen) the targeted pathologic condition or disorder, or one or more symptom associated therewith. Similarly, "responsive to" or "responds" and analogous terms refer to indications that the targeted pathological condition, or one or more symptom associated thereof, is prevented, ameliorated or lessened. The terms are also used herein to denote delaying the onset of, inhibiting (e.g. reducing or arresting the growth of), alleviating the effects of, or prolonging the life of a patient suffering from a disease, in particular a myeloproliferative disease, or indications that such markers have been accomplished. Those in need of treatment include those diagnosed with the disorder, those suspected of having the disorder, those predisposed to have the disorder as well as those in whom the disorder is to be prevented. Hence, the mammal to be treated herein may have been diagnosed as having the disorder or may be predisposed or susceptible to the disorder.

"Response" or "responsive" refers to a PBMC or a subject showing at least one altered characteristic subsequent to treatment. The altered characteristic of the subject may be amelioration or slowing down of the targeted pathologic condition or disorder.

As used herein, the terms "prevent", "preventing" and "prevention" refer to the prevention of the occurrence and/or recurrence or onset of one or more symptoms of a cancer disease in a subject resulting from the administration of a prophylactic or therapeutic agent.

The means and methods provided herein are mostly described for primary hematopoietic cells, or all monocyte cells. As the skilled person understands, primary hematopoietic cells comprise, inter alia, PBMCs and bone-marrow cells. Accordingly, the means and methods provided herein, which are described for PBMCs, are also disclosed for bone-marrow cells, as well as any other mono-nucleated cell.

"Therapeutic agents" within the meaning of the invention are molecules including, without limitation, polypeptides, peptides, glycoproteins, nucleic acids, synthetic and natural drugs, peptoides, polyenes, macrocyles, glycosides, terpenes, terpenoids, aliphatic and aromatic compounds, and their derivatives. In a preferred embodiment, the therapeutic agent is a chemical compound such as a synthetic and natural drug. In another preferred embodiment, the therapeutic agent effects amelioration and/or cure of a disease, disorder, pathology, and/or the symptoms associated therewith. The polymers may encapsulate one or more therapeutic agents or test compounds to be screened in the methods of the invention.

Suitable therapeutic agents include, without limitation, those presented in Goodman and Oilman's The Pharmacological Basis of Therapeutics (e.g., 9th Ed.) or The Merck Index (e.g., 12th Ed.). Genera of therapeutic agents include, without limitation, drugs that influence inflammatory responses, drugs that affect the composition of body fluids, drugs affecting electrolyte metabolism, chemotherapeutic agents (e.g., for hyperproliferative diseases, particularly cancer, for parasitic infections, and for microbial diseases), antineoplastic agents, immunosuppressive agents, drugs affecting the blood and blood-forming organs, hormones and hormone antagonists, vitamins and nutrients, vaccines, oligonucleotides and gene therapies. It will be understood that compositions comprising combinations, e.g. mixtures or blends of two or more active agents, such as two drugs, are also encompassed by the invention.

In one embodiment the therapeutic agent may be a drug or prodrug, antibody or vaccine. The method of the invention may be used to assess whether administration of a therapeutic agent to a patient triggers a response to the therapeutic agent, or a component of a delivery vehicle, excipient, carrier etc. administered with the therapeutic agent.

The precise nature of the therapeutic agent is not limiting to the invention. In non-limiting embodiments the method of the invention may be used to assess response to synthetic small molecules, naturally occurring substances, naturally occurring or synthetically produced biological agents, or any combination of two or more of the foregoing, optionally in combination with excipients, carriers or delivery vehicles.

The term "diagnosis" (along with grammatical variations thereof such as "diagnosing" or "diagnostic") refers to the identification of a molecular or pathological state, disease or condition, such as the identification of cancer, or refers to the identification of a cancer patient who may benefit from a particular treatment regimen.

The term "prognosis" (and grammatical variations thereof such as "prognosing" or "prognostic") refers to the prediction of the likelihood of benefit from a treatment such as a cancer therapy.

The term "prediction" or "predicting" is used herein to refer to the likelihood that a patient will respond either favorably or unfavorably to a particular therapeutic agent. In one embodiment, prediction or predicting relates to the extent of those responses. In one embodiment, the prediction or predicting relates to whether and/or the probability that a patient will survive or improve following treatment, for example treatment with a particular therapeutic agent, and for a certain period of time without disease progression.

Unless otherwise defined, all technical and scientific terms used herein have the same meaning as commonly understood by one of ordinary skill in the art to which this invention pertains. Although methods and materials similar or equivalent to those described herein can be used in the practice or testing of the present invention, suitable methods and materials are described below. In case of conflict, the present specification, including definitions, will control. In addition, the materials, methods, and examples are illustrative only and not intended to be limiting.

The general methods and techniques described herein may be performed according to conventional methods well known in the art and as described in various general and more specific references that are cited and discussed throughout the present specification unless otherwise indicated. See, e.g., Sambrook et al., Molecular Cloning: A Laboratory Manual, 2d ed., Cold Spring Harbor Laboratory Press, Cold Spring Harbor, N.Y. (1989) and Ausubel et al., Current Protocols in Molecular Biology, Greene Publishing Associates (1992), and Harlow and Lane Antibodies: A Laboratory Manual, Cold Spring Harbor Laboratory Press, Cold Spring Harbor, N.Y. (1990).

While aspects of the invention are illustrated and described in detail in the drawings and foregoing description, such illustration and description are to be considered illustrative or exemplary and not restrictive. It will be understood that changes and modifications may be made by those of ordinary skill within the scope and spirit of the following claims. In particular, the present invention covers further embodiments with any combination of features from different embodiments described above and below.

The invention also covers all further features shown in the figures individually, although they may not have been described in the previous or following description. Also, single alternatives of the embodiments described in the figures and the description and single alternatives of features thereof can be disclaimed from the subject matter of the other aspect of the invention.

Furthermore, in the claims the word "comprising" does not exclude other elements or steps, and the indefinite article "a" or "an" does not exclude a plurality. A single unit may fulfil the functions of several features recited in the claims. The terms "essentially", "about", "approximately" and the like in connection with an attribute or a value particularly also define exactly the attribute or exactly the value, respectively. Any reference signs in the claims should not be construed as limiting the scope.

The patent or application file contains at least one drawing executed in color. Copies of this patent or patent application publication with color drawing(s) will be provided by the Office upon request and payment of the necessary fee.

The present invention is also illustrated in some aspects by the following figures.

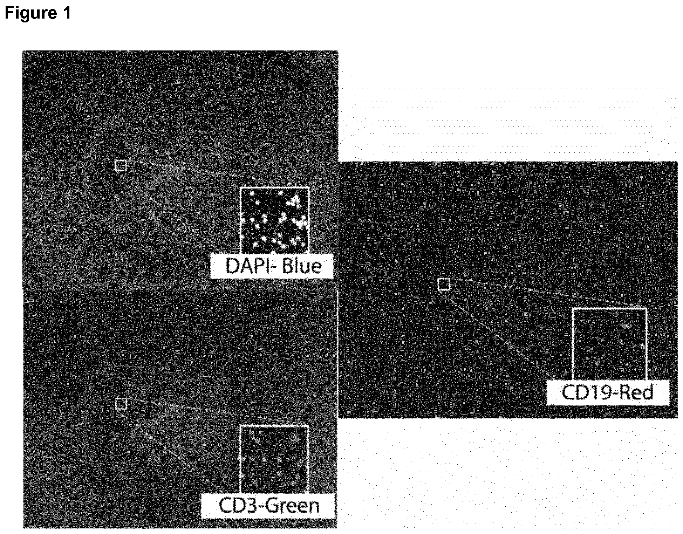

**Figure 1:**    Example of three color, 10x, image of the PBMC monolayer from a 384 well plate, split into three channels.

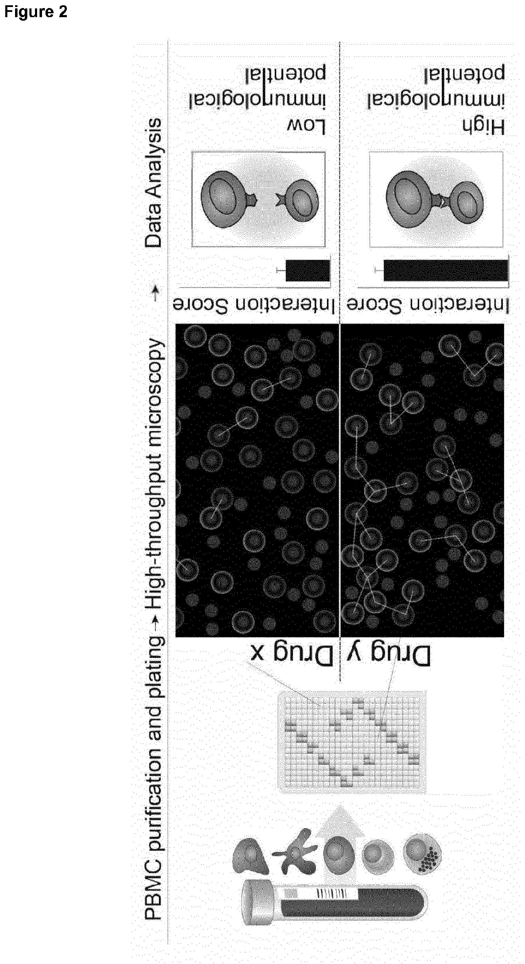

**Figure 2:**    Setup of the invention and cartoon explanation of the measurement of the propensity of cells to interact, and the relationship to the immunological potential.

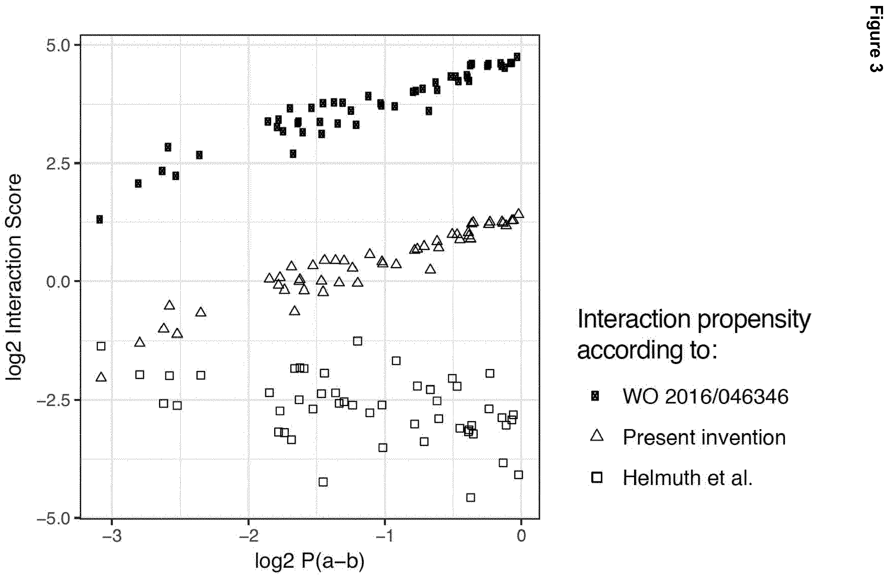

**Figure 3:**    Measured interaction propensity in synthetic datasets comprising of cells of type A, B, and C as a function of true interaction propensity P(a-b) between A and B cells as determined following WO 2016/046346, the present invention and Helmuth et al. in situations where cells interact with not more than one other partner.

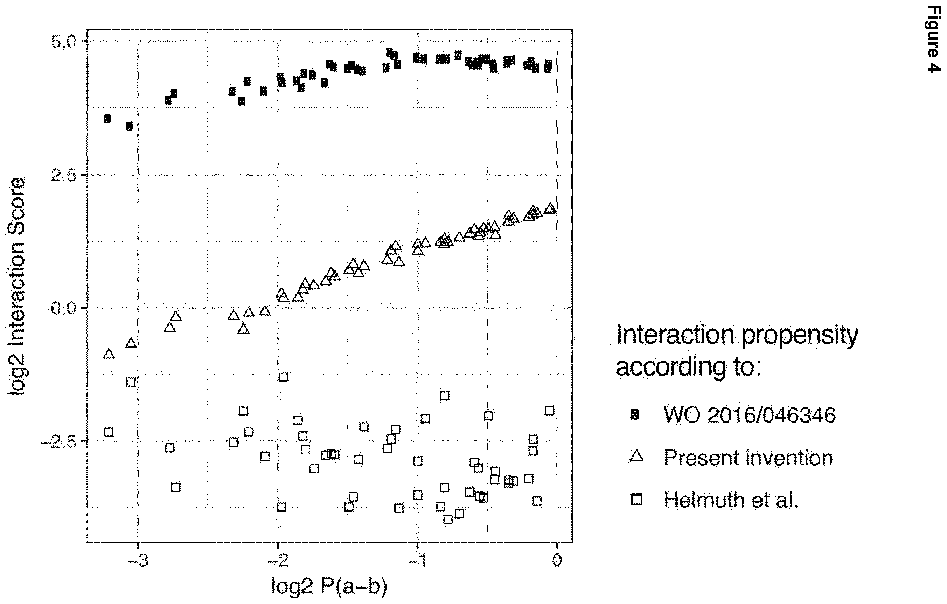

**Figure 4:**    Measured interaction propensity in synthetic datasets comprising of cells of type A, B, and C as a function of true interaction propensity P(a-b) between A and B cells as determined following WO 2016/046346, the present invention and Helmuth et al. in situations where cells interact with more than one other partner.

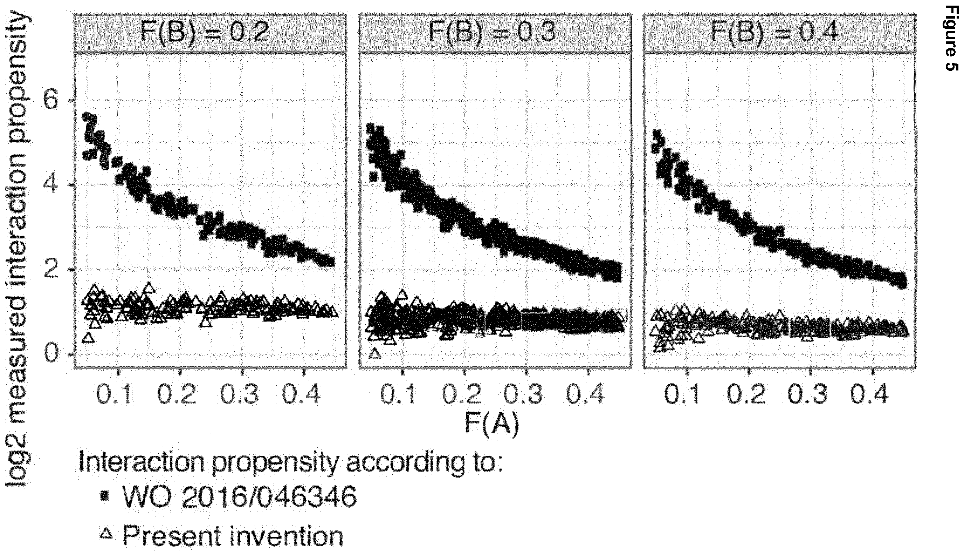

**Figure 5:**    Measured interaction propensity in synthetic datasets comprising of cells of type A, B, and C as a function of the fraction of A and B cells F(A) and F(B) of total cells as determined following WO 2016/046346, the present invention and Helmuth et al. in situations where cells interact with more than one other partner.

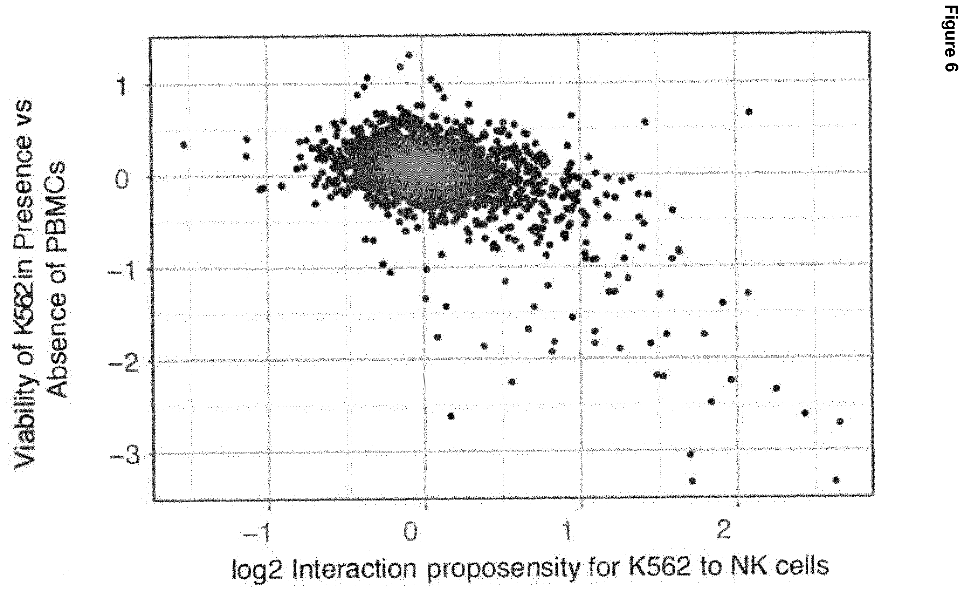

**Figure 6:**    Using the interaction propensity between K562 and NK cells determined using the present invention to identify modulators of NK cell mediated killing of K562 tumor cells.

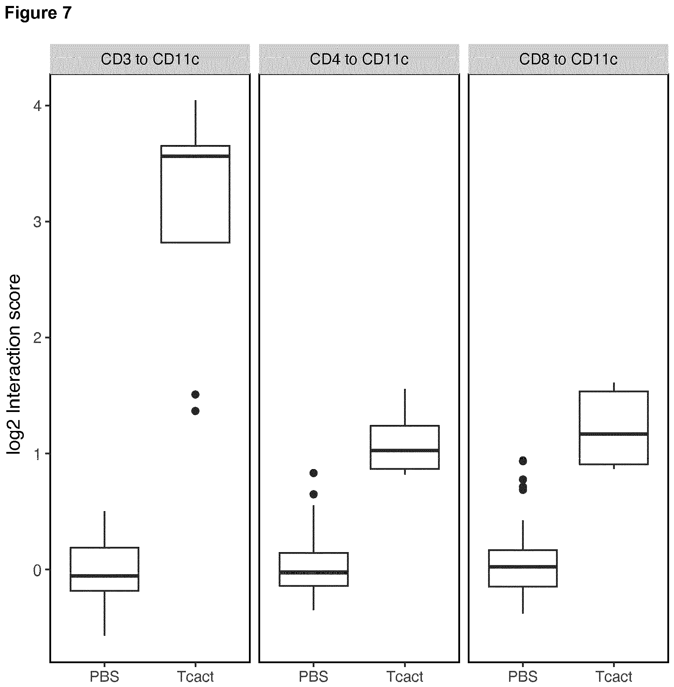

**Figure 7:**    Measured interaction propensity expressed as Log2 interaction score of T-cell subsets (CD4 or CD8+) to dendritic cells (CD11c+) upon activation with a T-cell directed ligand. PBS is the control, or reference, the Tcact is the T-cell activator.

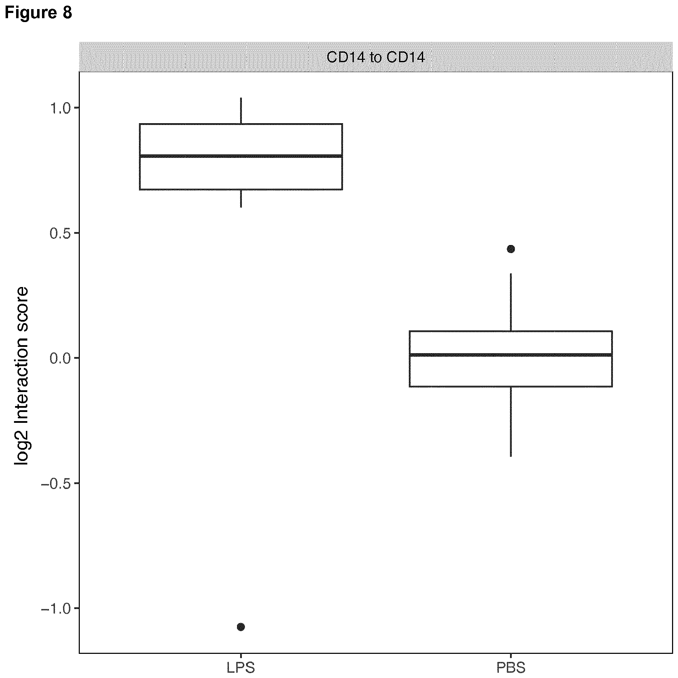

**Figure 8:**    Measured interaction propensity expressed as Log2 interaction score of CD14+ monocytes to each other after stimulation with gram negative endotoxin, LPS (lipopolysaccharide), which is known to induce such a monocyte clustering. Reference is PBS control.

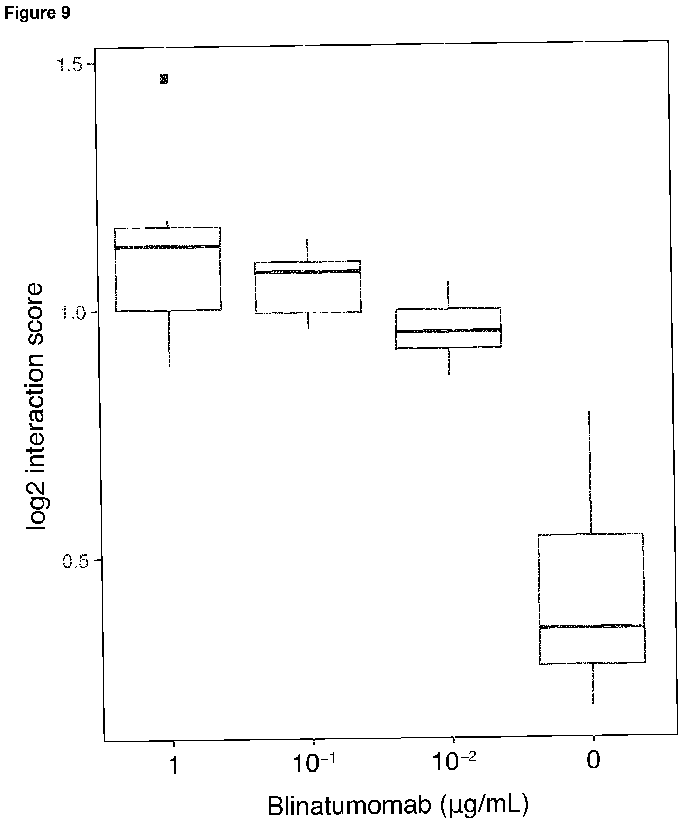

**Figure 9:**    Measured interaction propensity expressed as Log2 interaction score of CD3 cells to CD20 cells with increasing concentrations of the clinically approved biologic drug Blinatumomab, with media alone as a control (0). Blinatumomab has a known mechanism of action bringing together cytotoxic or other T-cells and cancerous B-cells, through this interaction, inducing cancer cell killing and thus reducing the cancerous burden.

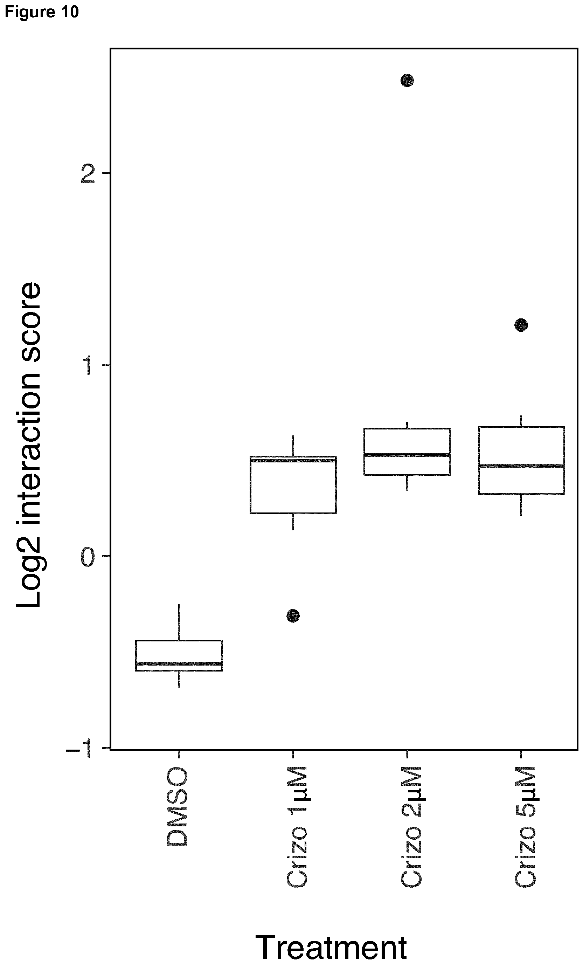

**Figure 10:**    Measured interaction propensity expressed as Log2 interaction score of CD11c and CD3 cells after treatment with the small molecule Crizotinib. Crizotinib, an ALK inhibitor, is approved for non-small cell lung cancer, and prior to the invention of the interaction score, did not have an immunomodulatory component to its mechanism of action, which was identified during a screening campaign

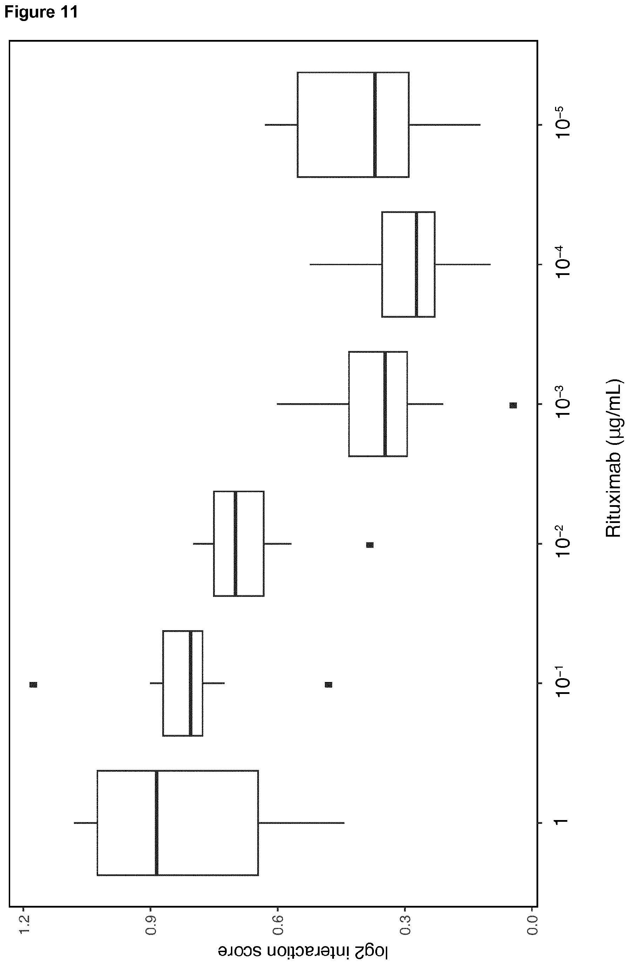

**Figure 11:**    Measured interaction propensity expressed as Log2 interaction score of CD19 cells to CD56 cells with increasing concentrations of the clinically approved biologic drug Rituximab, with media alone as a control (0). Rituximab has a known mechanism of action bringing together NK-cells and cancerous B-cells, and through this interaction inducing cancer , reducing the cancerous burden.

Aspects of the present invention are additionally described by way of the following illustrative non-limiting examples that provide a better understanding of embodiments of the present invention and of its many advantages. The following examples are included to demonstrate preferred embodiments of the invention. It should be appreciated by those of skill in the art that the techniques disclosed in the examples which follow represent techniques used in the present invention to function well in the practice of the invention, and thus can be considered to constitute preferred modes for its practice. However, those of skill in the art should appreciate, in light of the present disclosure that many changes can be made in the specific embodiments which are disclosed and still obtain a like or similar result without departing from the spirit and scope of the invention.

Unless otherwise indicated, established methods of recombinant gene technology were used as described, for example, in Sambrook, Russell "Molecular Cloning, A Laboratory Manual", Cold Spring Harbor Laboratory, N.Y. (2001) which is incorporated herein by reference in its entirety.

A number of documents including patent applications, manufacturer's manuals and scientific publications are cited herein. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

**Example 1**

**Experiment:** To establish a culture protocol of PBMCs from healthy donors or patients which results in a stainable monolayer (single imageable field / imaging plane) for imaging in 384 well plates.

**Method:** PBMCs were cultured as per protocol invented for pharmacoscopy. In a first step, blood is collected from (a) healthy proband(s) or patient(s). Typically, the volume is between 9 to 500 ml and the blood is stored in an appropriate container containing EDTA or heparin. The blood sample is then mixed at a 1:1 ration with PBS buffer. 30 ml of the blood/PBS mixture are layered over a 15 ml lymphoprep density gradient in 50 ml tubes for purification. The tubes are spun at 2000 rpm for 30 min at room temperature without break (no breaking of the centrifuge). The buffy coat above the density gradient and below the plasma is removed and placed into another 50 ml tube. Usually, the removed volume varies between 10 to 15 ml. The tube is then filled with PBS to 50 ml final volume and again spun at 2000 rpm for 5 min with centrifuge break. The supernatant is removed and the pellet suspended in RPMI with 20 ml 10% FCS and appropriate antibiotics. The pellet should have no more than a 5 mm thick band of RBCs. The cells are then counted to $4 \times 10^5$/ml and 50 $\mu$l are plated at a density of 20000 cells/well in corning 384-well imaging plates with black walls. The cells are left at room temperature for 10 to 15 min to settle and are then placed in a 37°C + 5% $CO_2$ incubator. The plates are then incubated for given time, ideally not more than overnight. If a viability dye is added, 30 $\mu$l of the supernatant are removed carefully by hand or with robotics and 30 $\mu$l of a 1:1000 mix in PBS of Invitrogen live/dead fixable 488 dye is added for 30 min at room temperature. Viability dye is removed as initial supernatant with automated pipet or robot. Disturbance of the monolayer should be avoided at this step. If no viability dye is added or immediately after it has been added, 30 $\mu$l of 2% formaldehyde with 0,1 % triton x-114 is added and the plates are incubated at room temperature for 15 min. The supernatant is removed (all of it) by flicking. Because the monolayer is already fixed at this stage, this will not disrupt the monolayer. For staining, 30 $\mu$l of antibody staining are added. Tested cocktails are a dilution of 1:300 of GFP, PE or APC labeled antibodies used for flow cytometry. The dilution allows avoidance of washing steps. The plates are incubated for 1 hour at room temperature. The antibody is removed by flicking as above and a 1:100 dilution of DAPI in PBS in 50 $\mu$l is added. The plates are stored at 4°C until imaging. Imaging is done at room temperature using an automated confocal microscope (PerkenElmer Operetta) with 4 non-overlapping channels and the data is exported for analysis.

**Results:** After culturing PBMCs utilizing our novel protocol, adherent and non-adherent PBMCs formed a monolayer that could be imaged in a single plane of view, utilizing an automated confocal microscope, allowing for automated drug screening minimized into 384-well plates. Microscopy confirmed that 20.000 cells ($\pm$5%) could be imaged using the newly developed method, termed Pharmacoscopy (Figure 1).

**Example 2**

**Experiment:** This example was done to test whether the methods of the present invention provide more reliable results than methods of the prior art, in particular methods provided in WO 2016/046346 or Helmuth et. al. (2010) BMC Bioinformatics.

**Methods:** Synthetic data sets were generated corresponding to cell mixtures comprising cells of type A, B, and C systematically changing the probability of cells of type A to interact with cells of type B and keeping the number of A, B and C cells in the mixture, and the probability of C cells to interact with B cells fixed. The propensity of A cells to interact with B cells was determined according to WO 2016/046346, Helmuth et. al. (2010) BMC Bioinformatics and the present invention.

**Results:** In situations where A cells interact with at maximum one B or C cell, the interaction propensity according to WO 2016/046346 and the present invention both show a linear relationship to the logarithm of the probability of A cells to interact with B cells (Figure 3). However, when cells of type A interact with more than one B or C cell (i.e., rosettas are formed), the interaction propensity determined using the method described in WO 2016/046346 fail to show a linear relationship with the logarithm of the probability of A cells to interact with B cells but flattens off at increasing interaction probabilities whereas the present invention still maintains this linear relationship (Figure 4). The interaction score according to Helmuth et al. fails to show a linear relationship with the logarithm of the probability of A cells to interact with B cells and is in fact not correlated to the probability of A and B cells to interact at all..

Measuring cell interaction propensities according to the present invention thus has a higher dynamic range in cases where cells can interact with more than one other cell.

This is commonly observed in cultures of primary immune cells where multiple cells of one type often interact with one cell of another type. This phenomenon is often described as the formation of "rosettas".

**Example 4**

**Experiment:** Synthetic data sets were generated corresponding to cell mixtures comprising cells of type A, B and C systematically changing the fraction of A, B and C cells in the mixture and keeping the probability of A and C cells to interact with B cells constant.

**Methods:** The propensity of A cells to interact with B cells was determined according to WO 2016/046346 and the present invention. Under the used simulation settings, the interaction propensity between A and B cells should ideally be invariant to changes in the fraction of A and C cells.

**Results:** While the interaction propensity determined according to the present invention was invariant to changes in the fraction of A and C cells, the interaction propensity according to WO 2016/046346 was not (Figure 5). Under many experimental setting changes in the fraction of A, B and C cells e.g., in response to exposure with a stimulus is not a confounding factor as the fraction of A, B and C cells can be assumed to be constant and the stimulus mostly affects change in the expression of cell surface molecules (e.g., up regulation of MHC on DCs increases the propensity of DCs and T-cells to interact). However, under settings where fractions of A and C cells may change such as in a situation where an immunological effector cells attack cancer or pathogen infected cells, having an interaction measure that is robust towards changes in the fraction of A cells is essential.

**Example 5**

In order to identify small molecule chemical entities that could modulate the ability of natural killer (NK) cells to kill tumor cells human peripheral blood mononuclear cells (PBMCs) and K562 human tumor cells were incubated at a density of approx. 4000 cells / mm2 and a ratio of 3:1 in the presence of different chemical compounds. As a control, the same absolute number of K562 cells was incubated in the presence of the same set of chemical compounds. At a set time, cells were fixed and permeabilised by the addition of formaldehyde and Triton X-114 and stained with antibodies against CD56 and DAPI. Microscopic images of the cells were taken and NK cells were identified by their positive staining for CD56 and K562 cells by their distinctive nuclear size and texture under DAPI staining using a neural network trained on 15,000 example cell images. The interaction propensity between K562 and NK cells was calculated and compared to the viability of K562 in the presence and absence of PBMCs relative to negative DMSO control. It was shown that the interaction propensity between K562 and NK cells was directly related to the viability of K562 in the presence and absence of PBMCs relative to negative DMSO control demonstrating that the interaction propensity as determined using the present invention allows the identification of molecules that modulate NK cell mediated killing of tumor cells (Figure 6).

**Example 6:**

Example relates to the effect of biologicals on the interaction score measured by this invention.

T-cells and APCs such as CD11c positive cells interact more frequently upon activation of T cells with standard activation cocktail consisting of anti CD3, anti CD28 antibodies and IL2.

PBMC monolayers prepared according as above were treated with a mixture of anti CD3 and anti CD28 antibodies plus 500 IU/mL IL2 for 48h. Monolayer were fixed and stained with different combinations of anti CD3, 4, 8 and 11c antibodies and the interaction propensity between cells measured using the present invention.

T-cell activation leads to an increase in measured interaction score between CD3, 4 or 8, and CD11c positive cells thus validating the approach (Figure 7).

**Example 7:**

Example relates to the effect of a biological macromolecule on the interaction score measured by this invention.

CD14 positive monocytes cluster upon treatment with LPS.

PBMC monolayers prepared according above were treated with 10 ng/mL LPS for 48h. Monolayer were fixed and stained with CD14 antibodies and the interaction propensity between cells measured using the present invention.

Treatment with LPS leads to a visible increase in clustering between CD14 positive cells which is reflected in an increase in measured interaction score between CD14 positive cells thus validating the approach (Figure 8).

**Example 8:**

**Objective:** The example relates to the determination of the effect of a biological agent that is known to induce the interaction between two cell types on the measured interaction propensity.

**Methods:** Blinatomumab is a bispecific antibody in BiTE format that cross links B and T cells inducing T cells to kill B cells.

PBMC monolayers were provided as described in WO 2016/046346, treated with blinatumomab or PBS control (0) for 48 h, fixed, stained with fluorescently labeled CD3 and CD19 antibodies and imaged using automated confocal microscopy. Subsequently, the interaction propensity of B (CD19 positive) and T cells (CD3 positive) in the provided non-adherent cell monolayers was determined using the present invention.

**Results:** Addition of blinatomumab exhibits an increase in interaction propensity between B and T cells over PBS control (0) thus validating the approach. Figure 9.

**Example 9:**

The example relates to the diagnosis of a disease.

Rheumatoid arthritis, and the severity of rheumatoid arthritis, can be diagnosed by quantifying the interaction propensity between activated B-cells (CD80+ and CD19+) to Th17 T-cells (CD3+ and CD28+) within the synovial fluid or peripheral blood. The closer the interactions of these activated cells, the higher the chance of a positive rheumatoid arthritis diagnosis.

Chronic reactive arthritis, under the disease category of secondary sterile inflammation post bacterial infection, can be diagnosed by quantifying the interaction of monocytes (CD14+) and T-cells (CD3+) within the synovial fluid, after clearance of the bacterial pathogen.

Atherosclerosis can be diagnosed by determining the spatial interaction of inflammatory resident monocytes (CCR2-, CD16+) within the blood to patient endothelial cells (CCR5+).

**Example 10:**

The example relates to the assessment and detection of the immunomodulation ability of a small molecule drug. Ciizotinib is approved for non-small cell lung cancer with no known immunomodulation properties.

PBMCs of a healthy donor are treated with Crizotinib or DMSO control at various concentrations and the interaction propensity of CD11C and CD3 cells in non adherent cell monolayers obtained from treated and untreated combined sample according to WO 2016/046346 determined using the present invention.

Crizotinib has an immomodulatory propensity to increase the interaction of DCs and T-cells as indicated by the interaction score; this interaction may be an unknown factor in the drug's clinical mechanism of action (Figure 10).

**Example 11:**

**Objective:** The example relates to the determination of the effect of a biological agent that is known to induce the interaction between two cell types on the measured interaction propensity.

**Methods:** Rituximab is an anti-CD20 antibody that links B and NK cells, bringing them into contact and inducing NK cells to kill B cells.

PBMC monolayers were provided as described in WO 2016/046346, treated with rituximab or PBS control (0) for 48 h, fixed, stained with fluorescently labeled CD56 and CD19 antibodies and imaged using automated confocal microscopy. Subsequently, the interaction propensity of B (CD19 positive) and NK cells (CD56 positive) in the provided non-adherent cell monolayers was determined using the present invention.

**Results:** Addition of blinatomumab exhibits an increase in interaction propensity between B and NK cells over PBS control (0) thus validating the approach (Figure 11).

**Example 12:**

**Objective:** The example relates to the determination if a patient diagnosed with a disease will respond to treatment, and if a biological mechanism of action can be assessed.

**Methods:** Rituximab is an anti-CD20 antibody that links B and NK cells inducing NK cells to kill B cells.

Bone marrow of a patients with B-cell acute lymphoblastic leukemia (B-ALL) and PBMCs of the same patient are combined, treated with Rituximab or PBS control and the interaction propensity of B and NK cells in non-adherent cell monolayers obtained from treated and untreated combined sample according to WO 2016/046346 determined using the present invention.

**Results:** Patients responding to Rituximab exhibit an increase in interaction propensity between B and NK cells whereas patients not responding clinically do not exhibit this increase in interaction.

**Example 13:**

**Objective:** The example relates to the determination if a patient diagnosed with a disease will respond to treatment, and if a biological mechanism of action can be assessed.

**Methods:** Blinatomumab is a bispecific antibody in BiTE format that cross links B and T cells inducing T cells to kill B cells.

Bone marrow of a patients with B-cell acute lymphoblastic leukemia (B-ALL) and PBMCs of the same patient are combined, treated with blinatumomab or PBS control and the interaction propensity of B and T cells in non-adherent cell monolayers obtained from treated and untreated combined sample according to WO 2016/046346 determined using the present invention.

**Results:** Patients responding to blinatumomab exhibit an increase in interaction propensity between B and T cells whereas patients not responding clinically do not exhibit this increase in interaction.

**Claims**

1. Method for determining the propensity of cells to interact in a population of cells comprising at least two distinguishable subpopulations of cells, wherein the method comprises the following steps:

   (a) determination of the number of interactions between cells of a first distinguishable subpopulation and cells of a second distinguishable subpopulation;
   (b) determination of the number of interactions in the population of cells by randomly assigning cells comprised in the population of cells to first and second distinguishable subpopulation of cells, wherein the absolute number of cells in the first and second distinguishable subpopulation of cells, respectively, are identical to the number of cells in the first and second distinguishable subpopulation of cells in (a); and
   (c) determination of the propensity of cells to interact by dividing (a) through (b), wherein propensity increases with the resulting number.

2. A population of cells obtained from a cell donor for use in determining whether the cell donor suffers from a disease or is predisposed to suffer from a disease, wherein the determination comprises the determination of the propensity of cells in the population to interact, wherein the population comprises at least two distinguishable subpopulations of cells and wherein the method comprises the following steps:

   (a) determination of the number of interactions between cells of a first distinguishable subpopulation and cells of a second distinguishable subpopulation;
   (b) determination of the number of interactions in the population of cells by randomly assigning cells comprised in the population of cells to first and second distinguishable subpopulation of cells, wherein the absolute number of cells in the first and second distinguishable subpopulation of cells, respectively, are identical to the number of cells in the first and second distinguishable subpopulation of cells in (a);
   (c) determination of the propensity of cells to interact by dividing (a) through (b), wherein propensity increases with the resulting number; and
   (d) determination whether the cell donor has a disease or is predisposed to have a disease based on the propensity of cells to interact.

3. A method for diagnosing a disease or predisposition to a disease of a cell donor, wherein the method comprises the determination of the propensity of cells to interact in a population of cells obtained from the donor, wherein the population of cells comprises at least two distinguishable subpopulations of cells and wherein the method comprises the following steps:

   (a) determination of the number of interactions between cells of a first distinguishable subpopulation and cells of a second distinguishable subpopulation;
   (b) determination of the number of interactions in the population of cells by randomly assigning cells comprised in the population of cells to first and second distinguishable subpopulation of cells, wherein the absolute number of cells in the first and second distinguishable subpopulation of cells, respectively, are identical to the number of cells in the first and second distinguishable subpopulation of cells in (a);
   (c) determination of the propensity of cells to interact by dividing (a) through (b), wherein propensity increases with the resulting number; and
   (d) determination whether the cell donor suffers from a disease or is predisposed to suffer from a disease based on the propensity of cells to interact.

4. A method for determining whether a subject suffering from or predisposed to a disease will respond or is responsive to treatment with a therapeutic agent by determining the alteration of the propensity of cells to interact in a population of cells obtained from the subject, wherein the population of cells comprises at least two distinguishable subpopulations of cells and wherein the method comprises the following steps:

(a) determination of the number of interactions between cells of a first distinguishable subpopulation and cells of a second distinguishable subpopulation;
(b) determination of the number of interactions in the cell sample by randomly assigning cells comprised in the population of cells to first and second distinguishable subpopulation of cells, wherein the absolute number of cells in the first and second distinguishable subpopulation of cells are identical to the number of cells in the first and second distinguishable subpopulation of cells in (a);
(c) determination of the propensity of cells to interact by dividing (a) through (b), wherein propensity increases with the resulting number; and
(d) determination of the alteration of the propensity of cells to interact by comparing the propensity prior to addition of a therapeutic agent and subsequent to addition of a therapeutic agent and thereby determining whether the subject will respond or is responsive to treatment with the therapeutic agent.

5. A population of cells obtained from a cell donor for use in a diagnostic method for determining whether a subject suffering from or predisposed to a disease will respond or is responsive to treatment with a therapeutic agent, wherein the determination comprises the determination of the propensity of cells in the monolayer to interact, wherein the monolayer comprises at least two distinguishable subpopulations of cells and wherein the method comprises the following steps:

(a) determination of the number of interactions between cells of a first distinguishable subpopulation and cells of a second distinguishable subpopulation;
(b) determination of the number of interactions in the cell sample by randomly assigning cells comprised in the cell sample to first and second distinguishable subpopulation of cells, wherein the absolute number of cells in the first and second distinguishable subpopulation of cells, respectively, are identical to the number of cells in the first and second distinguishable subpopulation of cells in (a);
(c) determination of the propensity of cells to interact by dividing (a) through (b), wherein propensity increases with the resulting number; and
(d) determination of the alteration of the propensity of cells to interact by comparing the propensity prior to addition of a therapeutic agent and subsequent to addition of a therapeutic agent and thereby determining whether the subject will respond or is responsive to treatment with the therapeutic agent.

6. A method for screening of a therapeutic agent by determining the alteration of the propensity of cells to interact in a population of cells due to addition of one or more test substance(s), wherein the population of cells comprises at least two distinguishable subpopulations of cells and wherein the method comprises the following steps:

(a) determination of the number of interactions between cells of a first distinguishable subpopulation and cells of a second distinguishable subpopulation;
(b) determination of the number of interactions in the cell sample by randomly assigning cells comprised in the cell sample to first and second distinguishable subpopulation of cells, wherein the absolute number of cells in the first and second distinguishable subpopulation of cells, respectively, are identical to the number of cells in the first and second distinguishable subpopulation of cells in (a);
(c) determination of the propensity of cells to interact by dividing (a) through (b), wherein propensity increases with the resulting number; and
(d) determination of the alteration of the propensity of cells to interact by comparing the propensity prior to addition of one or more test substance(s) and subsequent to addition of one or more test substance(s) and thereby determining whether the one or more test substance(s) qualify as therapeutic agent.

7. A population of cells for use in screening of a therapeutic agent by determining the alteration of the propensity of cells to interact in the population of cells due to addition of one or more test substance(s), wherein the population of cells comprises at least two distinguishable subpopulations of cells and wherein the method comprises the following steps:

(a) determination of the number of interactions between cells of a first distinguishable subpopulation and cells of a second distinguishable subpopulation;
(b) determination of the number of interactions in the cell sample by randomly assigning cells comprised in the cell sample to first and second distinguishable subpopulation of cells, wherein the absolute number of cells in the first and second distinguishable subpopulation of cells, respectively, are identical to the number of cells in the first and second distinguishable subpopulation of cells in (a);

(c) determination of the propensity of cells to interact by dividing (a) through (b), wherein propensity increases with the resulting number; and

(d) determination of the alteration of the propensity of cells to interact by comparing the propensity prior to addition of one or more test substance(s) and subsequent to addition of one or more test substance(s) and thereby determining whether the one or more test substance(s) qualify as therapeutic agent.

8. The method of any one of claims 1, 3, 4 or 6 or the population of cells of claims 2, 5 or 7, wherein first and second distinguishable subpopulations are identical.

9. The method of any one of claims 1, 3, 4, 6 or 8 or the population of cells of claims 2, 5, 7 or 8, wherein step (b) is repeated and the determined number of interactions is averaged, preferably wherein step (b) is repeated more than at least 1000 times.

10. The method of any one of claims 1, 3, 4, 6, 8 or 9 or the population of cells of claims 2, 5, 7, 8, 9, wherein the cells are (a) peripheral blood mononuclear cells (PBMCs) or (b) bone marrow cells.

11. The method of any one of claims 1, 3, 4, 6, 8, 9 or 10 or the population of cells of claims 2, 5, 7, 8, 9 or 10, wherein the disease is a myeloproliferative disorder, inflammatory disorder, latent virus infection, cellular growth disorder, cellular chemotaxis disorder, metabolic disorder or autoimmune disorder.

12. The method of claim 10 or the population of cells of claim 10, wherein the disease is leukemia or lymphoma.

**Figure 1**

DAPI- Blue

CD3-Green

CD19-Red

**Figure 2**

**Figure 3**

**Figure 4**

**Figure 5**

**Figure 6**

**Figure 7**

**Figure 8**

**Figure 9**

**Figure 10**

**Figure 11**

Europäisches
Patentamt

European
Patent Office

Office européen
des brevets

# EUROPEAN SEARCH REPORT

Application Number

EP 17 15 7806

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | HELMUTH JO A ET AL: "Beyond co-localization: inferring spatial interactions between sub-cellular structures from microscopy images", BMC BIOINFORMATICS, BIOMED CENTRAL, LONDON, GB, vol. 11, no. 1, 7 July 2010 (2010-07-07), page 372, XP021071699, ISSN: 1471-2105, DOI: 10.1186/1471-2105-11-372 * the whole document * | 1-12 | INV. G01N33/569 G01N33/574 |
| A | AFENYA EVANS K ET AL: "Mathematical modeling of bone marrow - peripheral blood dynamics in the disease state based on current emerging paradigms, part I", MATHEMATICAL BIOSCIENCES, vol. 274, 2016, pages 83-93, XP029461088, ISSN: 0025-5564, DOI: 10.1016/J.MBS.2016.01.010 * the whole document * | 1-12 | |
| X,D | WO 2016/046346 A1 (CEMM FORSCHUNGSZENTRUM FÜR MOLEKULARE MEDIZIN GMBH [AT]) 31 March 2016 (2016-03-31) * the whole document * | 1-12 | **TECHNICAL FIELDS SEARCHED (IPC)** G01N |
| T | GREGORY I VLADIMER ET AL: "Global survey of the immunomodulatory potential of common drugs", NATURE CHEMICAL BIOLOGY, vol. 13, no. 6, 24 April 2017 (2017-04-24) , pages 681-690, XP055376766, Basingstoke ISSN: 1552-4450, DOI: 10.1038/nchembio.2360 | | |

The present search report has been drawn up for all claims

| Place of search | Date of completion of the search | Examiner |
|---|---|---|
| Munich | 30 May 2017 | Moreno de Vega, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 17 15 7806

This annex lists the patent family members relating to the patent documents cited in the above-mentioned European search report.
The members are as contained in the European Patent Office EDP file on
The European Patent Office is in no way liable for these particulars which are merely given for the purpose of information.

30-05-2017

| Patent document cited in search report | Publication date | Patent family member(s) | Publication date |
|---|---|---|---|
| WO 2016046346 A1 | 31-03-2016 | AU 2015323725 A1<br>SG 11201702316S A<br>WO 2016046346 A1 | 13-04-2017<br>27-04-2017<br>31-03-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2016046346 A
- US 6207299 B
- US 6322901 B
- US 6576291 B
- US 6649138 B
- US 6682596 B
- US 6815064 B
- US 20050012182 A
- WO 2005001889 A

**Non-patent literature cited in the description**

- **SNIJDER et al.** *Nature Reviews,* 2011, vol. 12, 119
- **SLACK et al.** *PNAS,* 2008, vol. 105 (49), 19306-11
- **HELMUTH et al.** *BMC Bioinformatics,* 2010, vol. 11, 372
- **HALLEK et al.** *Blood,* 2008, vol. 111 (12
- **PANDA, S. ; RAVINDRAN, B.** Isolation of Human PBMCs. *Bio-protocol,* 2013, vol. 3 (3), e323
- **CHAN et al.** *J. Immunol. Methods,* 2013, vol. 388 (1-2), 25-32
- **QU et al.** Alternative Routes toward High Quality CdSe Nanocrystals. *Nano Lett.,* 2001, vol. 1 (6), 333-337
- Goodman and Oilman's The Pharmacological Basis of Therapeutics
- The Merck Index
- **SAMBROOK et al.** Molecular Cloning: A Laboratory Manual. Cold Spring Harbor Laboratory Press, 1989
- **AUSUBEL et al.** Current Protocols in Molecular Biology. Greene Publishing Associates, 1992
- **HARLOW ; LANE.** Antibodies: A Laboratory Manual. Cold Spring Harbor Laboratory Press, 1990
- **SAMBROOK ; RUSSELL.** Molecular Cloning, A Laboratory Manual. Cold Spring Harbor Laboratory, 2001
- **HELMUTH.** *BMC Bioinformatics,* 2010
- **HELMUTH.** *BMC Bioinformatics and the present invention,* 2010